(19)

Europäisches
Patentamt
European
Patent Office
Office européen
des brevets

(11)     **EP 3 973 978 A1**

(12)     **EUROPEAN PATENT APPLICATION**

(43)  Date of publication:
   **30.03.2022   Bulletin 2022/13**

(21)  Application number: **21196665.0**

(22)  Date of filing: **14.09.2021**

(51) International Patent Classification (IPC):
   ***A61K 38/16*** (2006.01)      ***A61K 38/35*** (2006.01)
   ***A61P 37/00*** (2006.01)

(52) Cooperative Patent Classification (CPC):
   **A61K 38/35; A61K 38/16; A61P 37/00**

(84)  Designated Contracting States:
   **AL AT BE BG CH CY CZ DE DK EE ES FI FR GB
   GR HR HU IE IS IT LI LT LU LV MC MK MT NL NO
   PL PT RO RS SE SI SK SM TR**
   Designated Extension States:
   **BA ME**
   Designated Validation States:
   **KH MA MD TN**

(30)  Priority:  **25.09.2020   US 202063083532 P
        29.06.2021   US 202117362497**

(71)  Applicant: **Mallinckrodt Ard IP Unlimited Company
   Dublin D15 TX2V (IE)**

(72)  Inventor: **WRIGHT, Dale
   Dublin, D15 TX2V (IE)**

(74)  Representative: **Jones, Nicholas Andrew
   Withers & Rogers LLP
   2 London Bridge
   London SE1 9RA (GB)**

(54)     **METHODS OF MODULATING CORTICOSTEROID RESPONSE**

(57)     The present disclosure is directed to methods of modulating corticosteroid responses in subjects in need thereof.

EP 3 973 978 A1

SEQUENCE LISTING

<110> MALLINCKRODT ARD IP UNLIMITED COMPANY
     WRIGHT, Dale
     SHARMA, Prabha
     GALEN, Karen
     AWEIFEL, Ben
     FITCH, Rick

<120> METHODS OF MODULATING CORTICOSTEROID RESPONSE

<130> H-AD-00013T.1 WO/US

<150> US 62/794,369
<151> 2019-01-18

<150> PCT/US2020/014444
<151> 2020-01-21

<150> US 63/083,532
<151> 2020-09-25

<160> 1

<170> PatentIn version 3.5

<210> 1
<211> 39
<212> PRT
<213> Porcine

<400> 1

Ser Tyr Ser Met Glu His Phe Arg Trp Gly Lys Pro Val Gly Lys Lys
1               5                   10                  15

Arg Arg Pro Val Lys Val Tyr Pro Asp Gly Ala Glu Asp Gln Leu Ala
            20                  25                  30

Glu Ala Phe Pro Leu Glu Phe
            35

**Description**

**CROSS REFERENCE TO RELATED APPLICATIONS**

This application, which is a continuation-in-part application, claims priority to PCT Application No. PCT/US2020/014444, filed January 21, 2020, which claims priority to U.S. Provisional Application No. 62/794,369 filed January 18, 2019, and this application claims priority to U.S. Provisional Application No. 63/083,532, filed September 25, 2020, the disclosure of each of which is incorporated herein by reference.

**FIELD OF THE DISCLOSURE**

The present disclosure is directed to methods of modulating corticosteroid responses in subjects in need thereof.

**BACKGROUND OF THE DISCLOSURE**

Diseases and disorders characterized by excessive inflammation are often treated by generating a corticosteroid response in the subject. Such treatments, however, may cause unnecessary or even undesirable side effects. There is a need in the art, therefore, for methods of tailoring a corticosteroid response in a subject to result in more efficient treatment of inflammation and inflammatory disorders.

**BRIEF DESCRIPTION OF THE FIGURES**

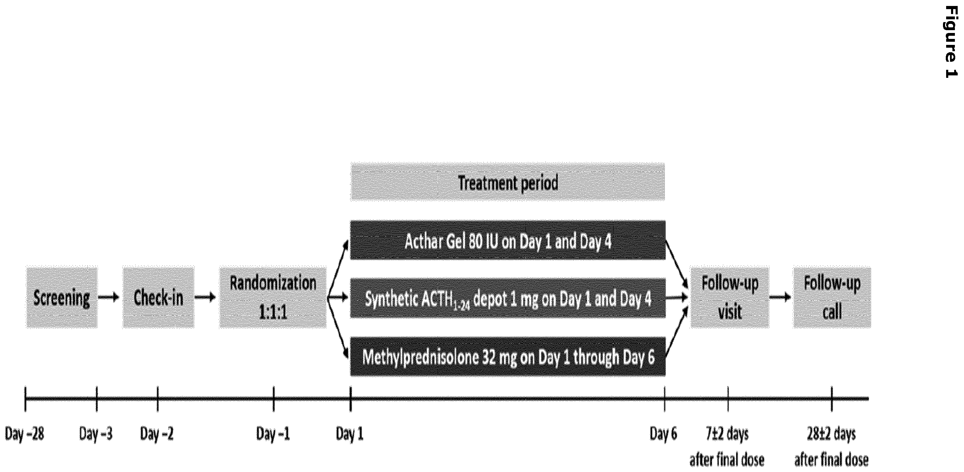

**FIG. 1** depicts a schematic of the study design.

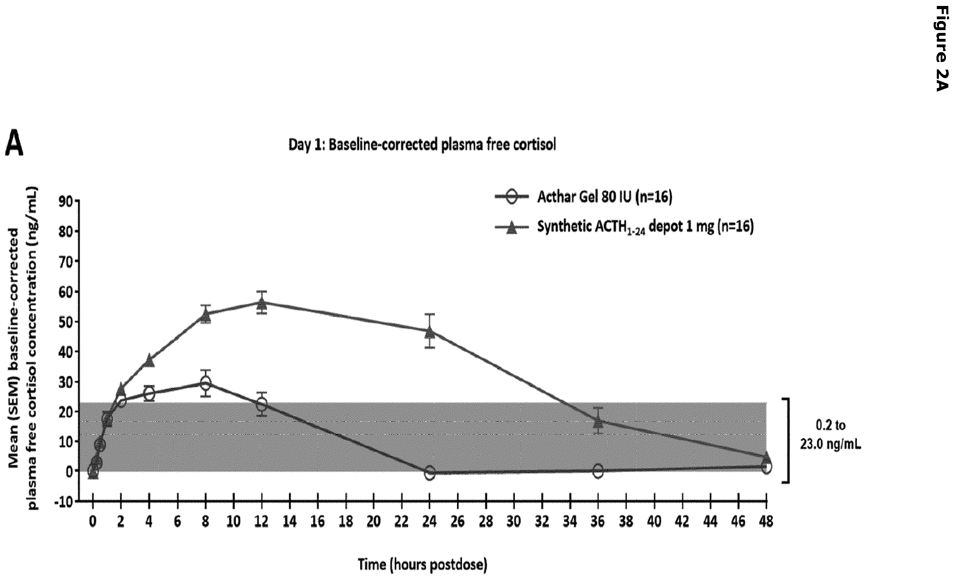

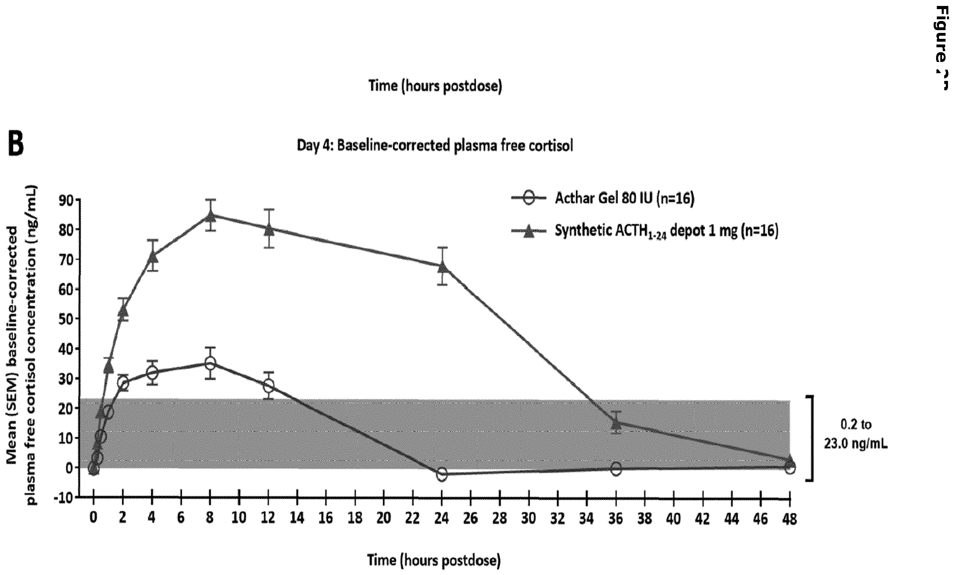

**FIG. 2A and 2B** depict graphs showing the mean (SEM) baseline-corrected plasma free cortisol concentrations after administration of Acthar Gel and synthetic $ACTH_{1-24}$ depot on study Day 1 (Fig. 2A) and Day 4 (Fig. 2B). The range for baseline plasma free cortisol concentration observed for all study drugs is represented by the gray shaded area (0.2-23.0 ng/mL). Baseline-corrected free cortisol concentrations were obtained by subtracting the time-matched baseline (Day -1) free cortisol concentrations from the respective Day 1 and Day 4 free cortisol concentrations for each study drug. Abbreviations: $ACTH_{1-24}$, the first 24 amino acids of adrenocorticotropic hormone; SEM, standard error of the mean.

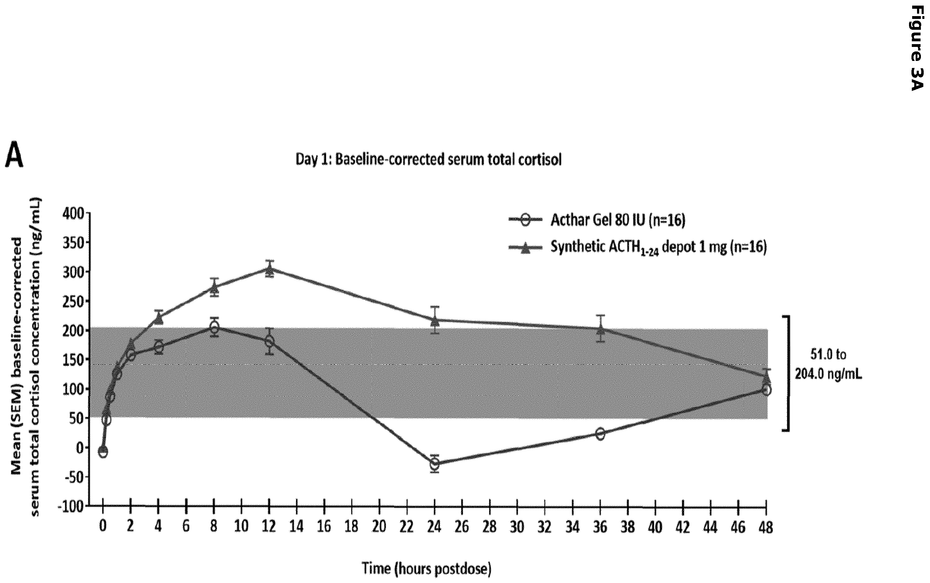

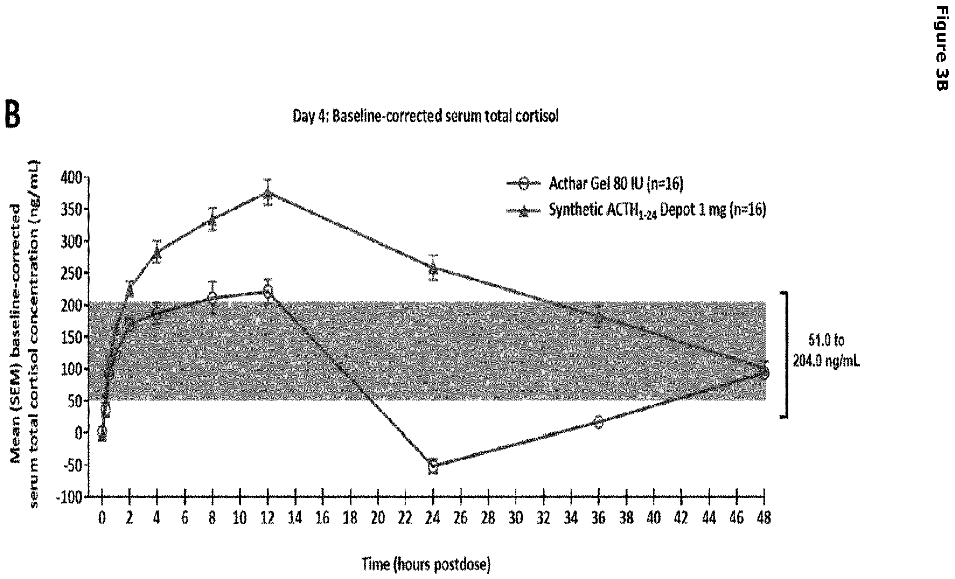

**FIG. 3A and 3B** depict graphs showing the mean (SEM) baseline-corrected serum total cortisol concentrations after administration of Acthar Gel and synthetic $ACTH_{1-24}$ depot on study Day 1 (Fig. 3A) and Day 4 (Fig. 3B). The range for baseline serum total cortisol concentration observed for all study drugs is represented by the gray shaded area (51.0 to 204.0 ng/mL). Baseline-corrected total cortisol concentrations were obtained by subtracting the time-matched baseline (Day -1) total cortisol concentrations from the respective Day 1 and Day 4 total cortisol concentrations for each study drug. Abbreviations: $ACTH_{1-24}$, the first 24 amino acids of adrenocorticotropic hormone; SEM, standard error of the mean.

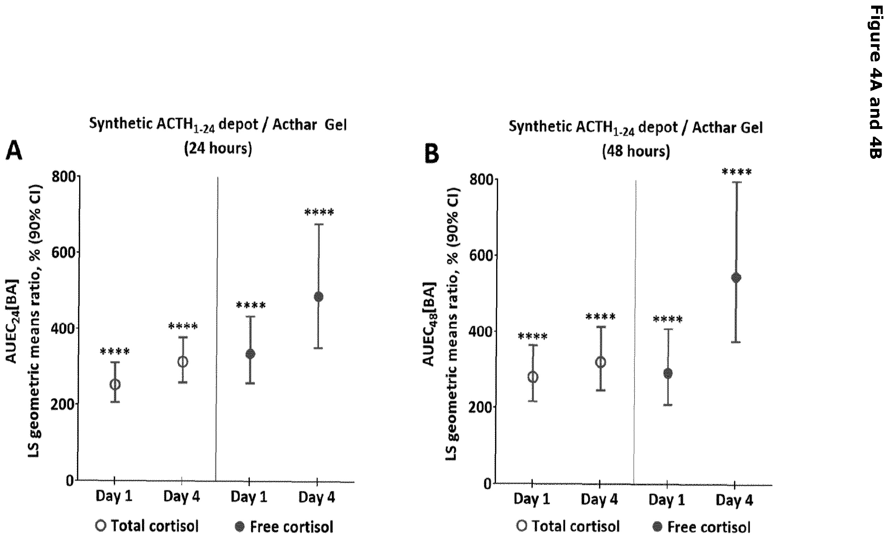

**FIG. 4A and 4B** depict graphs showing a comparison of serum total and plasma free cortisol $AUEC_{24}[BA]$ (Fig. 4A) and $AUEC_{48}[BA]$ (Fig. 4B) using the LS geometric mean ratio (percentage) of synthetic $ACTH_{1-24}$ depot to Acthar Gel. Data points labeled with **** indicate $p<0.0001$ for the LS geometric means ratio (percentage) of synthetic $ACTH_{1-24}$ depot to Acthar Gel for each day using ANOVA models, with study drug as a fixed effect and subject as a random effect. Abbreviations: $ACTH_{1-24}$, the first 24 amino acids of adrenocorticotropic hormone; ANOVA, analysis of variance; $AUEC_{24}[BA]$, baseline-corrected area under the effect curve for the concentration-time profile from time 0 to 24 hours; $AUEC_{48}[BA]$, baseline-corrected area under the effect curve for the concentration-time profile from time 0 to 48 hours; CI, confidence interval; LS, least squares.

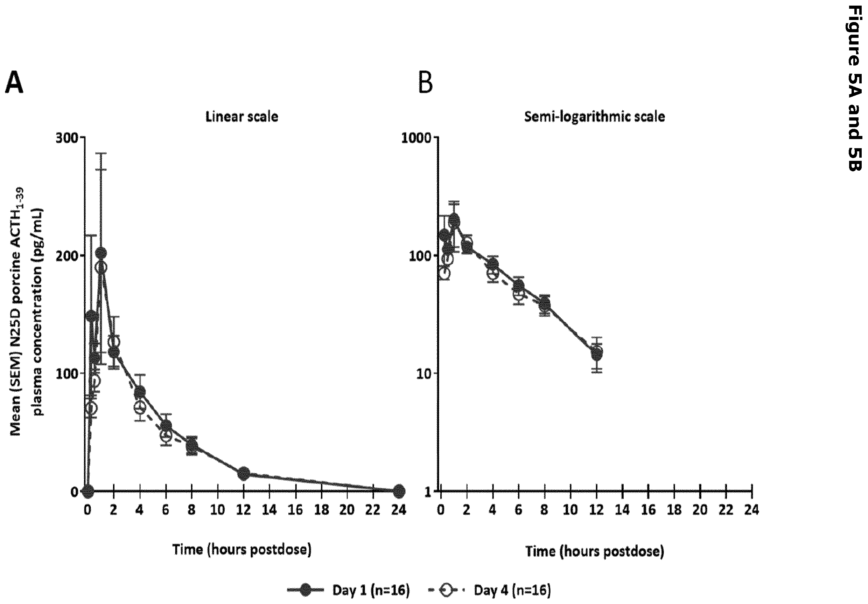

**FIG. 5A and 5B** depicts graphs showing mean (SEM) plasma N25D porcine $ACTH_{1-39}$ concentration-time profile on study Day 1 and Day 4 using a linear scale (left; Figure 5A) and a semi-logarithmic scale (right; Figure 5B). Abbreviations: $ACTH_{1-39}$, the first 39 amino acids of adrenocorticotropic hormone; N25D, N-25 deamidated; SEM, standard error of the mean.

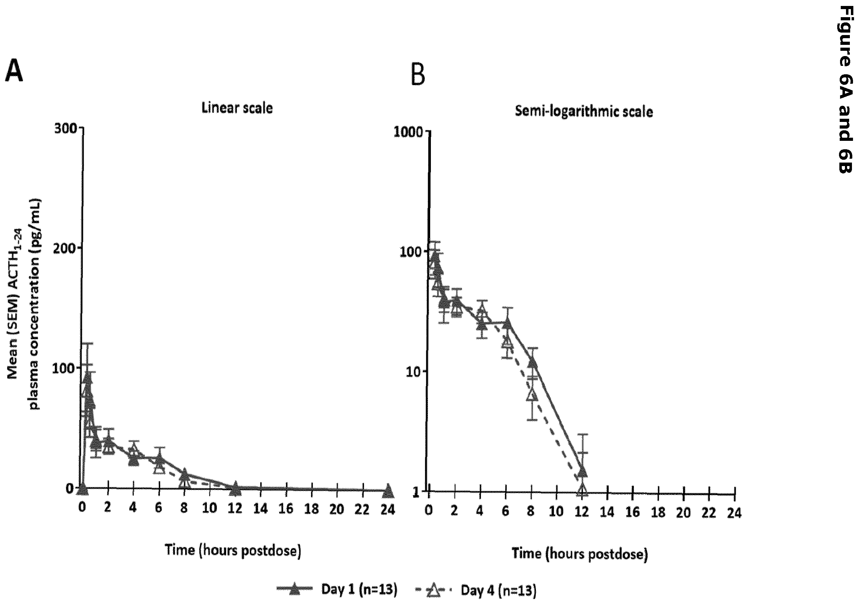

**FIG. 6A and 6B** depicts graphs showing mean (SEM) plasma $ACTH_{1-24}$ concentration-time profile on study Day 1 and Day 4 using a linear scale (left; Figure 6A) and a semi-logarithmic scale (right; Figure 6B). Abbreviations: $ACTH_{1-24}$, the first 24 amino acids of adrenocorticotropic hormone; SEM, standard error of the mean.

**SUMMARY OF THE DISCLOSURE**

One aspect of the present disclosure encompasses a method of choosing an adrenal corticotropin treatment for a subject in need of such treatment. The method comprises (a) determining if a delayed corticosteroid response or a non-delayed response is appropriate for the subject; and (b) administering an adrenal corticotropin treatment consisting essentially of an RCI dose if a non-delayed response is appropriate or administering an adrenal corticotropin treatment consisting essentially of a synthetic adrenal corticotropin composition if a delayed response is appropriate.

Another aspect of the present disclosure encompasses a method of designing a corticosteroid response in a subject. The method comprises (a) selecting a subject in need of a designed corticosteroid response, (b) designing an appropriate corticosteroid response for the subject, and (c) administering one or more doses of an adrenal corticotropin treatment to the subject at a dose sufficient to invoke the desired corticosteroid response.

Yet another aspect of the present disclosure encompasses a method of treating a subject. The method comprises: (a) determining if a strong corticosteroid response in the subject is beneficial; (b) when the answer to step (a) is no, administering one or more doses of repository corticotropin injection (RCI) to the subject at a dose sufficient to not invoke a strong corticosteroid response in the subject, wherein the desired corticosteroid response aids in the treatment of the subject.

Other aspects and iterations of the present disclosure are detailed below.

## DETAILED DESCRIPTION

The present disclosure encompasses methods of treating a subject in need of treatment. Generally speaking, the methods comprise administering an adrenal corticotropin treatment to tailor a specific corticosteroid response in the subject.

## I. TREATMENTS

Methods of the present disclosure encompass the use and administration of adrenal corticotropin treatment(s) to a subject. Such treatments are described below.

Generally speaking, an adrenal corticotropin treatment refers to a treatment that mimics the physiological activity of adrenocorticotropic hormone (ACTH). ACTH is a 39 amino acid peptide hormone that is secreted by the pituitary gland and is a part of the hypothalamus-pituitary-adrenal (HPA) axis that maintains the stress response and homeostasis in the body. Physiologically, the principal effects of ACTH are stimulation of the adrenal cortex with subsequent increased production of glucocorticosteroids and/or cortisol from the adrenal cortex. ACTH levels are tightly regulated in the body via a negative feedback loop wherein glucocorticosteroids suppress the release of corticotropin release hormone (CRH) from the pituitary and CRH-mediated release of ACTH. In some instances, cortisol helps restore homeostasis after stress. In some instances, changed patterns of serum cortisol levels are observed in connection with abnormal ACTH levels. In some instances, prolonged ACTH-mediated secretion of abnormal levels of cortisol (e.g., higher or lower levels of cortisol compared to cortisol levels in normal individuals) has detrimental effects. Thus, any perturbation in the levels of ACTH has profound physiological implications.

In vivo, ACTH is synthesized from a precursor polypeptide pre-pro-opiomelanocortin (pre-POMC). The removal of the signal peptide during translation produces a 267 amino acid polypeptide POMC. POMC undergoes a series of post-translational modifications to yield various polypeptide fragments including and not limited to ACTH, $\beta$-lipotropin, $\gamma$-lipotropin, $\alpha$, $\beta$, $\gamma$-Melanocyte Stimulating Hormone (MSH) and $\beta$-endorphin. POMC, ACTH and $\beta$-lipotropin are also secreted from the pituitary gland in response to the hormone corticotropin-releasing hormone (CRH). The first 13 amino acids of $ACTH_{1-39}$ are cleaved to form $\alpha$-melanocyte-stimulating hormone ($\alpha$-MSH).

In some instances, an abnormality in ACTH levels is associated with inflammation (e.g., increased release of pro-inflammatory cytokines). In some instances, an abnormality in ACTH levels is associated with reduced VEGF secretion. In some instances, reduced VEGF secretion is associated with reduced growth of new blood vessels and inadequate oxygen supply to tissues (e.g., neurons and/or muscles).

In some embodiments of the use or methods described herein, the ACTH peptide is a $ACTH_{1-39}$ peptide having the formula:

H-Ser-Tyr-Ser-Met-Glu-His-Phe-Arg-Trp-Gly-

1  2  3  4  5  6  7  8  9  10

Lys-Pro-Val-Gly-Lys-Lys-Arg-Arg-Pro-Val-

11  12  13  14  15  16  17  18  19  20

Lys-Val-Tyr-Pro-Asp-Gly-Ala-Glu-Asp-Gln-

21  22  23  24  25  26  27  28  29  30

Leu-Ala-Glu-Ala-Phe-Pro-Leu-Glu-Phe-OH

31  32  33  34  35  36  37  38  39

or a fragment thereof, a variant thereof or any combination thereof.

In some embodiments, an adrenal corticotropin treatment may comprise a synthetic adrenal corticotropin composition. In other embodiments, an adrenal corticotropin treatment may comprise a non-synthetic adrenal corticotropin composition. Both of these embodiments are described in more detail below.

### (a) synthetic adrenal corticotropin composition

As used herein, the phrase "synthetic adrenal corticotropin composition" refers to a composition comprising an ACTH peptide, homolog, variant, aggregate, complex, preparation, or prodrug that has been recombinantly produced or otherwise synthesized. The phrase "synthetic adrenal corticotropin composition" excludes compositions comprising an ACTH peptide, homolog, variant, aggregate, complex, or preparation naturally, endogenously, produced in a cell of an organism.

A synthetic adrenal corticotropin composition may include corticotropin, tetracosactide or the like. In some embodiments, a synthetic adrenal corticotropin composition may include any synthetically produced ACTH peptide, ACTH fragment, or ACTH preparation as described herein.

The term "ACTH peptide" refers to $ACTH_{1-39}$ peptide. The term "ACTH peptide homolog" includes ACTH peptide or peptide fragments or ACTH-like compounds with about 40%, about 45%, about 50%, about 55%, about 60%, about 65%, about 70%, about 75%, about 80%, about 85%, about 90%, or about 95% sequence identity with $ACTH_{1-39}$.

As used herein the term "variant" may mean a peptide or polypeptide that differs in amino acid sequence by the insertion, deletion, or conservative substitution of amino acids, but retains at least one biological activity. A variant may also mean a protein with an amino acid sequence that is substantially identical to a referenced protein with an amino acid sequence that retains at least one biological activity. A conservative substitution of an amino acid, i.e., replacing an amino acid with a different amino acid of similar properties (e.g., hydrophilicity, degree and distribution of charged regions) is recognized in the art as typically involving a minor change. These minor changes can be identified, in part, by considering the hydropathic index of amino acids, as understood in the art. Kyte et al., J. Mol. Biol. 157:105-132 (1982). The hydropathic index of an amino acid is based on a consideration of its hydrophobicity and charge. It is known in the art that amino acids of similar hydropathic indexes can be substituted and still retain protein function. In one aspect, amino acids having hydropathic indexes of $\pm 2$ are substituted. The hydrophilicity of amino acids can also be used to reveal substitutions that would result in proteins retaining biological function. A consideration of the hydrophilicity of amino acids in the context of a peptide permits calculation of the greatest local average hydrophilicity of that peptide, a useful measure that has been reported to correlate well with antigenicity and immunogenicity. U.S. Patent No. 4,554,101, incorporated fully herein by reference. Substitution of amino acids having similar hydrophilicity values can result in peptides retaining biological activity, as is understood in the art. Substitutions may be performed with amino acids having hydrophilicity values within $\pm 2$ of each other. Both the hyrophobicity index and the hydrophilicity value of amino acids are influenced by the particular side chain of that amino acid. Consistent with that observation, amino acid substitutions that are compatible with biological function are understood to depend on the relative similarity of the amino acids, and particularly the side chains of those amino acids, as revealed by the hydrophobicity, hydrophilicity, charge, size, and other properties.

The phrase "ACTH peptide, fragment, variant, or prodrug" also includes, in certain embodiments, pre-POMC, POMC, β-lipotropin, γ-lipotropin, Melanocyte Stimulating Hormone (α-MSH, β-MSH, γ-MSH), β-endorphin, or the like, or any other polypeptide fragment that is a post-translational product of the POMC gene. POMC genes for various species are found in the NCBI GenBank including and not limited to human POMC transcript variant 1, mRNA, (NCBI Accession number NM_001035256), human POMC transcript variant 2, mRNA, (NCBI Accession number NM_000939), swine pro-opiomelanocortin, mRNA (NCI Accession number S73519), swine proopiomelanocortin protein (POMC) gene (NCBI Accession number EU184858), rat proopiomelanocortin (POMC) gene (NCBI Accession number K01877), or the like. Other examples of POMC genes include, for example, catfish POMC gene described in Animal Genetics, 2005, 36, 160-190. Melanocortin peptides, including ACTH and alpha, beta and gamma MSH derive from post-translational modification of POMC. A number of melanocortin peptides share an invariant sequence of four amino acids, His-Phe-Arg-Trp, which also correspond to residues 6-9 of ACTH and alpha-MSH. Accordingly, also contemplated within the scope of embodiments presented herein, is the use of amino acid sequences that correspond to alpha MSH, beta MSH or gamma MSH. See Catania et al., Pharmacol. Rev. 2004, 56: 1-29.

The term "ACTH aggregate" refers to a physical grouping of peptides which may comprise ACTH peptide, or fragments, analogs or homologs thereof. Such an aggregate may comprise hydrogen-bonded molecules and/or molecules held by bridging interactions via, for example, a salt bridge, a metal ion, and the like.

The term "ACTH complex" refers to ACTH or fragments or variants thereof that are optionally complexed with other proteins (e.g., Bovine Serum Albumin), or metal ions, or charged polymers (e.g., polylysine), or fragments, homologs or analogs of ACTH, or any other suitable complexes that retain the functional characteristics of ACTH or ACTH fragments or analogs thereof and/or allow for formulation of ACTH or ACTH fragments or analogs thereof into suitable dosage forms.

In some embodiments, a synthetic adrenal corticotropin composition comprises an ACTH preparation. As used herein, "ACTH preparation" refers to a mixture containing ACTH peptide and/or other peptide fragments and/or other proteins and/or other substances that together form a composition that is suitable for any methods and/or dosing regimen described herein.

Synthetic adrenal corticotropin compositions are available commercially. For instance, Synacthen®, Adrenomone®, or the like. Further examples of commercially available synthetic adrenal corticotropin compositions may include and are not limited to Adrenocorticotropic Hormone (ACTH) (1-10) (human), Adrenocorticotropic Hormone (ACTH) (1-13) (human), Adrenocorticotropic Hormone (ACTH) (1-16) (human), Adrenocorticotropic Hormone (ACTH) (1-17) (human), Adrenocorticotropic Hormone (ACTH) (1-24) (human), Adrenocorticotropic Hormone (ACTH) (1-39) (human), Adrenocorticotropic Hormone (ACTH) (1-39) (rat), Adrenocorticotropic Hormone (ACTH) (18-39) (human), Adrenocorticotropic Hormone (ACTH) (4-10) (human), Adrenocorticotropic Hormone (ACTH) (1-4), Adrenocorticotropic Hormone (ACTH) (1-14) or the like available from, for example, GenScript.

### (b) non-synthetic adrenal corticotropin composition

In certain embodiments of the present disclosure, an adrenal corticotropin treatment may comprise administration of a non-synthetic adrenal corticotropin composition. In these embodiments, ACTH is obtained from a cell that naturally, endogenously, produces an ACTH peptide, homolog, variant, aggregate, complex, preparation, or prodrug. For instance, a non-synthetic adrenal corticotropin composition may be prepared from a homogenized pituitary extract of an appropriate animal (e.g., pituitary extract of a pig). Any suitable method may be used to obtain a homogenized pituitary extract. In some embodiments, a homogenized pituitary extract includes ACTH peptide and/or other peptide fragments and/or other proteins and/or other substances that are contemplated as being part of the ACTH preparation that is compatible with any method described herein.

In one aspect, a non-synthetic adrenal corticotropin composition encompasses a repository corticotropin injection. An example of a repository corticotropin injection is Acthar® Gel (previously referred to as H.P. Acthar® Gel). Acthar® Gel is a naturally sourced complex mixture of adrenocorticotropic hormone analogs and other pituitary peptides. The Acthar® Gel manufacturing process converts the initial porcine pituitary extract with low ACTH content into a mixture having modified porcine ACTH and other related peptide analogs solubilized in gelatin. A major component in the formulated complex mixture is N-25 deamidated porcine ACTH (1-39). Acthar® Gel is supplied as a sterile preparation in 16% gelatin to provide a prolonged release after intramuscular or subcutaneous injection. Acthar® Gel also contains 0.5% phenol, not more than 0.1% cysteine (added), sodium hydroxide and/or acetic acid to adjust pH and water for injection.

In particular embodiments, a non-synthetic adrenal corticotropin composition of the present disclosure refers to a composition comprising N-25 deamidated porcine ACTH (1-39). For instance, in some embodiments, a non-synthetic adrenal corticotropin composition of the present disclosure is a naturally sourced complex mixture comprising N-25 deamidated porcine ACTH (1-39).

In certain embodiments, a repository corticotropin injection is administered intramuscularly. In some embodiments, the recommended regimen is a daily dose of 150 U/m2 (divided into twice daily intramuscular injections of 75 U/m2) administered over a 2-week period. Dosing can then be gradually tapered over a 2-week period to avoid adrenal insufficiency. The following is one suggested tapering schedule: 30 U/m2 in the morning for 3 days; 15 U/m2 in the morning for 3 days; 10 U/m2 in the morning for 3 days; and 10 U/m2 every other morning for 6-days.

In various embodiments, a respository corticotropin injection has a recommended dose for daily intramuscular or subcutaneous injections of 80-120 units for 2-3 weeks for acute exacerbations. In other embodiments, a repository corticotropin injection has a recommended dose of 40-80 units given intramuscularly or subcutaneously every 24-72 hours. Dosage should be individualized according to the medical condition of each patient. Frequency and dose of the drug should be determined by considering the severity of the disease and the initial response of the patient.

In embodiments where the repository corticotropin injection is Acthar® Gel, it should be noted that although drug dependence does not occur, sudden withdrawal of Acthar® Gel after prolonged use may lead to adrenal insufficiency or recurrent symptoms which make it difficult to stop the treatment. It may be necessary to taper the dose and increase the injection interval to gradually discontinue the medication.

In some embodiments, Acthar® Gel may be administered intramuscularly and a daily dose of 150 U/m2 (divided into twice daily intramuscular injections of 75 U/m$^2$) may be administered over a 2-week period. The dosing with Acthar® gel can be gradually tapered over a 2-week period to avoid adrenal insufficiency. In one exemplary embodiment, a tapering schedule is as follows: 30 U/m$^2$ in the morning for 3 days; 15 U/m$^2$ in the morning for 3 days; 10 U/m$^2$ in the morning for 3 days; and 10 U/m$^2$ every other morning for 6-days.

Acthar® gel is typically dosed based on body surface area (BSA). For calculation of body surface area, use the following formula:

$$BSA(m^2) = \sqrt{\frac{weight~(\text{kg}) \times height~(\text{cm})}{3600}}$$

In other embodiments, Acthar® Gel may be administered intramuscularly or subcutaneous at doses of 80-120 units for 2-3 weeks for acute exacerbations. In still other embodiments, the dose of Acthar® Gel may be 40-80 units given intramuscularly or subcutaneously every 24-72 hours. In some embodiments, dosing is individualized according to the medical condition of each patient. Frequency and dose of the drug can be determined by considering the severity of the disease and the initial response of the patient.

Acthar® Gel is contraindicated for intravenous administration. Acthar® gel is contraindicated where congenital infections are suspected in infants. Administration of live or live attenuated vaccines is contraindicated in patients receiving immunosuppressive doses of Acthar® Gel. Acthar® Gel is contraindicated in patients with scleroderma, osteoporosis, systemic fungal infections, ocular herpes simplex, recent surgery, history of or the presence of a peptic ulcer, congestive heart failure, uncontrolled hypertension, primary adrenocortical insufficiency, adrenocortical hyperfunction or sensitivity to proteins of porcine origin.

### (c) pharmaceutical compositions

The present disclosure also provides pharmaceutical compositions. A pharmaceutical composition comprises a synthetic adrenal corticotropin composition or a non-synthetic corticotropin composition. For instance, a pharmaceutical composition of the present disclosure may comprise a synthetic ACTH peptide, fragment, prodrug and variant, or any combination thereof, as an active ingredient, and at least one pharmaceutically acceptable excipient; alternatively, a pharmaceutical composition of the present disclosure may comprise a non-synthetic ACTH peptide, fragment, prodrug and variant, or any combination thereof, as an active ingredient, and at least one pharmaceutically acceptable excipient.

The pharmaceutically acceptable excipient may be a diluent, a binder, a filler, a buffering agent, a pH modifying agent, a disintegrant, a dispersant, a preservative, a lubricant, or a coloring agent. The amount and types of excipients utilized to form pharmaceutical compositions may be selected according to known principles of pharmaceutical science.

In each of the embodiments described herein, a composition of the present disclosure may optionally comprise one or more additional drug or therapeutically active agent in addition to the Adrenal corticotropin composition, prodrug and variant, or any combination thereof. Thus, in addition to the therapies described herein, one may also provide to the subject other therapies known to be efficacious for treatment of the disease, disorder, or condition. In some embodiments, the additional drug or therapeutically active agent is anti-inflammatory.

### (i) Diluent

In one embodiment, the excipient may be a diluent. The diluent may be compressible (i.e., plastically deformable) or abrasively brittle. Non-limiting examples of suitable compressible diluents include microcrystalline cellulose (MCC), cellulose derivatives, cellulose powder, cellulose esters (i.e., acetate and butyrate mixed esters), ethyl cellulose, methyl cellulose, hydroxypropyl cellulose, hydroxypropyl methylcellulose, sodium carboxymethylcellulose, corn starch, phosphated corn starch, pregelatinized corn starch, rice starch, potato starch, tapioca starch, starch-lactose, starch-calcium carbonate, sodium starch glycolate, glucose, fructose, lactose, lactose monohydrate, sucrose, xylose, lactitol, mannitol, malitol, sorbitol, xylitol, maltodextrin, and trehalose. Non-limiting examples of suitable abrasively brittle diluents include dibasic calcium phosphate (anhydrous or dihydrate), calcium phosphate tribasic, calcium carbonate, and magnesium carbonate.

*(ii) Binder*

In another embodiment, the excipient may be a binder. Suitable binders include, but are not limited to, starches, pregelatinized starches, gelatin, polyvinylpyrrolidone, cellulose, methylcellulose, sodium carboxymethylcellulose, ethylcellulose, polyacrylamides, polyvinyloxoazolidone, polyvinylalcohols, C12-C18 fatty acid alcohol, polyethylene glycol, polyols, saccharides, oligosaccharides, polypeptides, oligopeptides, and combinations thereof.

*(iii) Filler*

In another embodiment, the excipient may be a filler. Suitable fillers include, but are not limited to, carbohydrates, inorganic compounds, and polyvinylpyrrolidone. By way of non-limiting example, the filler may be calcium sulfate, both di- and tri-basic, starch, calcium carbonate, magnesium carbonate, microcrystalline cellulose, dibasic calcium phosphate, magnesium carbonate, magnesium oxide, calcium silicate, talc, modified starches, lactose, sucrose, mannitol, or sorbitol.

*(iv) Buffering Agent*

In still another embodiment, the excipient may be a buffering agent. Representative examples of suitable buffering agents include, but are not limited to, phosphates, carbonates, citrates, tris buffers, and buffered saline salts (e.g., Tris buffered saline or phosphate buffered saline).

*(v) pH Modifier*

In various embodiments, the excipient may be a pH modifier. By way of non-limiting example, the pH modifying agent may be sodium carbonate, sodium bicarbonate, sodium citrate, citric acid, or phosphoric acid.

*(vi) Disintegrant*

In a further embodiment, the excipient may be a disintegrant. The disintegrant may be non-effervescent or effervescent. Suitable examples of non-effervescent disintegrants include, but are not limited to, starches such as corn starch, potato starch, pregelatinized and modified starches thereof, sweeteners, clays, such as bentonite, micro-crystalline cellulose, alginates, sodium starch glycolate, gums such as agar, guar, locust bean, karaya, pecitin, and tragacanth. Non-limiting examples of suitable effervescent disintegrants include sodium bicarbonate in combination with citric acid and sodium bicarbonate in combination with tartaric acid.

*(vii) Dispersant*

In yet another embodiment, the excipient may be a dispersant or dispersing enhancing agent. Suitable dispersants may include, but are not limited to, starch, alginic acid, polyvinylpyrrolidones, guar gum, kaolin, bentonite, purified wood cellulose, sodium starch glycolate, isoamorphous silicate, and microcrystalline cellulose.

*(viii) Excipient*

In another alternate embodiment, the excipient may be a preservative. Non-limiting examples of suitable preservatives include antioxidants, such as BHA, BHT, vitamin A, vitamin C, vitamin E, or retinyl palmitate, citric acid, sodium citrate; chelators such as EDTA or EGTA; and antimicrobials, such as parabens, chlorobutanol, or phenol.

*(ix) Lubricant*

In a further embodiment, the excipient may be a lubricant. Non-limiting examples of suitable lubricants include minerals such as talc or silica; and fats such as vegetable stearin, magnesium stearate, or stearic acid.

*(x) Coloring Agent*

In still a further embodiment, the excipient may be a coloring agent. Suitable color additives include, but are not limited to, food, drug and cosmetic colors (FD&C), drug and cosmetic colors (D&C), or external drug and cosmetic colors (Ext. D&C).

The weight fraction of the excipient or combination of excipients in the composition may be about 99% or less, about 97% or less, about 95% or less, about 90% or less, about 85% or less, about 80% or less, about 75% or less, about 70% or less, about 65% or less, about 60% or less, about 55% or less, about 50% or less, about 45% or less, about 40% or less, about 35% or less, about 30% or less, about 25% or less, about 20% or less, about 15% or less, about 10% or less, about 5% or less, about 2%, or about 1% or less of the total weight of the composition.

The agents and compositions described herein can be formulated by any conventional manner using one or more pharmaceutically acceptable carriers or excipients as described in, for example, Remington's Pharmaceutical Sciences (A.R. Gennaro, Ed.), 21st edition, ISBN: 0781746736 (2005), incorporated herein by reference in its entirety. Such formulations will contain a therapeutically effective amount of a biologically active agent described herein, which can be in purified form, together with a suitable amount of carrier so as to provide the form for proper administration to the subject.

The term "formulation" refers to preparing a drug in a form suitable for administration to a subject, such as a human. Thus, a "formulation" can include pharmaceutically acceptable excipients, including diluents or carriers.

The term "pharmaceutically acceptable" as used herein can describe substances or components that do not cause unacceptable losses of pharmacological activity or unacceptable adverse side effects. Examples of pharmaceutically acceptable ingredients can be those having monographs in United States Pharmacopeia (USP 29) and National Formulary (NF 24), United States Pharmacopeial Convention, Inc, Rockville, Maryland, 2005 ("USP/NF"), or a more recent edition, and the components listed in the continuously updated Inactive Ingredient Search online database of the FDA. Other useful components that are not described in the USP/NF, etc. may also be used.

The term "pharmaceutically acceptable excipient," as used herein, can include any and all solvents, dispersion media, coatings, antibacterial and antifungal agents, isotonic, or absorption delaying agents. The use of such media and agents for pharmaceutical active substances is well known in the art (see generally Remington's Pharmaceutical Sciences (A.R. Gennaro, Ed.), 21st edition, ISBN: 0781746736 (2005)). Except insofar as any conventional media or agent is incompatible with an active ingredient, its use in the therapeutic compositions is contemplated. Supplementary active ingredients can also be incorporated into the compositions.

A "stable" formulation or composition can refer to a composition having sufficient stability to allow storage at a convenient temperature, such as between about 0 °C and about 60 °C, for a commercially reasonable period of time, such as at least about one day, at least about one week, at least about one month, at least about three months, at least about six months, at least about one year, or at least about two years.

The formulation should suit the mode of administration. The agents of use with the current disclosure can be formulated by known methods for administration to a subject using several routes which include, but are not limited to, parenteral, pulmonary, topical, intradermal, intramuscular, intraperitoneal, intravenous, subcutaneous, intranasal, epidural, ophthalmic, buccal, and rectal. The individual agents may also be administered in combination with one or more additional agents or together with other biologically active or biologically inert agents. Such biologically active or inert agents may be in fluid or mechanical communication with the agent(s) or attached to the agent(s) by ionic, covalent, Van der Waals, hydrophobic, hydrophilic or other physical forces.

Controlled-release (or sustained-release) preparations may be formulated to extend the activity of the agent(s) and reduce dosage frequency. Controlled-release preparations can also be used to effect the time of onset of action or other characteristics, such as blood levels of the agent, and consequently affect the occurrence of side effects. Controlled-release preparations may be designed to initially release an amount of an agent(s) that produces the desired therapeutic effect, and gradually and continually release other amounts of the agent to maintain the level of therapeutic effect over an extended period of time. In order to maintain a near-constant level of an agent in the body, the agent can be released from the dosage form at a rate that will replace the amount of agent being metabolized or excreted from the body. The controlled-release of an agent may be stimulated by various inducers, e.g., change in pH, change in temperature, enzymes, water, or other physiological conditions or molecules.

### (d) Administration

### (i) Dosage Forms

A composition of the present disclosure may be formulated into various dosage forms and administered by a number of different means that will deliver a therapeutically effective amount of the active ingredient.

In some embodiments, such compositions can be administered via inhalation, parenterally, topically, or in other suitable administration routes in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous, intravenous, intramuscular, intra-articular, or intrasternal injection, or infusion techniques. Formulation of drugs is discussed in, for example, Gennaro, A. R., Remington's Pharmaceutical Sciences, Mack Publishing Co., Easton, Pa. (18th ed, 1995), and Liberman, H. A. and Lachman, L., Eds., Pharmaceutical Dosage Forms, Marcel Dekker Inc., New York, N.Y. (1980). In a specific embodiment, a composition may be a cosmetic.

For embodiments where administration is by inhalation, the compounds are delivered in the form of an aerosol spray from pressured container or dispenser which contains a suitable propellant, e.g., a gas such as carbon dioxide, or a nebulizer.

For parenteral administration (including subcutaneous, intradermal, intravenous, intramuscular, intra-articular and intraperitoneal), the preparation may be an aqueous or an oil-based solution. Aqueous solutions may include a sterile diluent such as water, saline solution, a pharmaceutically acceptable polyol such as glycerol, propylene glycol, or other synthetic solvents; an antibacterial and/or antifungal agent such as benzyl alcohol, methyl paraben, chlorobutanol, phenol, thimerosal, and the like; an antioxidant such as ascorbic acid or sodium bisulfite; a chelating agent such as etheylenediaminetetraacetic acid; a buffer such as acetate, citrate, or phosphate; and/or an agent for the adjustment of tonicity such as sodium chloride, dextrose, or a polyalcohol such as mannitol or sorbitol. The pH of the aqueous solution may be adjusted with acids or bases such as hydrochloric acid or sodium hydroxide. Oil-based solutions or suspensions may further comprise sesame, peanut, olive oil, or mineral oil. The compositions may be presented in unit-dose or multi-dose containers, for example sealed ampoules and vials, and may be stored in a freeze-dried (lyophilized) condition requiring only the addition of the sterile liquid carrier for example water for injections, immediately prior to use. Extemporaneous injection solutions and suspensions may be prepared from sterile powders, granules, and tablets.

For topical (e.g., transdermal or transmucosal) administration, penetrants appropriate to the barrier to be permeated are generally included in the preparation. Pharmaceutical compositions adapted for topical administration may be formulated as ointments, creams, suspensions, lotions, powders, solutions, pastes, gels, sprays, aerosols, or oils. In some embodiments, the pharmaceutical composition is applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles, and mouth washes. Transmucosal administration may be accomplished through the use of nasal sprays, aerosol sprays, tablets, or suppositories, and transdermal administration may be via ointments, salves, gels, patches, or creams as generally known in the art.

In certain embodiments, a composition comprising an Adrenal corticotropin composition, prodrug, variant, or any combination thereof, is encapsulated in a suitable vehicle to either aid in the delivery of the compound to target cells, to increase the stability of the composition, or to minimize potential toxicity of the composition. As will be appreciated by a skilled artisan, a variety of vehicles are suitable for delivering a composition of the present disclosure. Non-limiting examples of suitable structured fluid delivery systems may include nanoparticles, liposomes, microemulsions, micelles, dendrimers, and other phospholipid-containing systems. Methods of incorporating compositions into delivery vehicles are known in the art.

In one alternative embodiment, a liposome delivery vehicle may be utilized. Liposomes, depending upon the embodiment, are suitable for delivery of the Adrenal corticotropin composition, prodrug, variant, or any combination thereof, in view of their structural and chemical properties. Generally speaking, liposomes are spherical vesicles with a phospholipid bilayer membrane. The lipid bilayer of a liposome may fuse with other bilayers (e.g., the cell membrane), thus delivering the contents of the liposome to cells. In this manner, an Adrenal corticotropin composition, prodrug, variant, or any combination thereof may be selectively delivered to a cell by encapsulation in a liposome that fuses with the targeted cell's membrane.

Liposomes may be comprised of a variety of different types of phosolipids having varying hydrocarbon chain lengths. Phospholipids generally comprise two fatty acids linked through glycerol phosphate to one of a variety of polar groups. Suitable phospholids include phosphatidic acid (PA), phosphatidylserine (PS), phosphatidylinositol (PI), phosphatidylglycerol (PG), diphosphatidylglycerol (DPG), phosphatidylcholine (PC), and phosphatidylethanolamine (PE). The fatty acid chains comprising the phospholipids may range from about 6 to about 26 carbon atoms in length, and the lipid chains may be saturated or unsaturated. Suitable fatty acid chains include (common name presented in parentheses) n-dodecanoate (laurate), n-tretradecanoate (myristate), n-hexadecanoate (palmitate), n-octadecanoate (stearate), n-eicosanoate (arachidate), n-docosanoate (behenate), n-tetracosanoate (lignocerate), cis-9-hexadecenoate (palmitoleate), cis-9-octadecanoate (oleate), cis,cis-9,12-octadecandienoate (linoleate), all cis-9, 12, 15-octadecatrienoate (linolenate), and all cis-5,8,11,14-eicosatetraenoate (arachidonate). The two fatty acid chains of a phospholipid may be identical or different. Acceptable phospholipids include dioleoyl PS, dioleoyl PC, distearoyl PS, distearoyl PC, dimyristoyl PS, dimyristoyl PC, dipalmitoyl PG, stearoyl, oleoyl PS, palmitoyl, linolenyl PS, and the like.

The phospholipids may come from any natural source, and, as such, may comprise a mixture of phospholipids. For example, egg yolk is rich in PC, PG, and PE, soy beans contains PC, PE, PI, and PA, and animal brain or spinal cord is enriched in PS. Phospholipids may come from synthetic sources too. Mixtures of phospholipids having a varied ratio of individual phospholipids may be used. Mixtures of different phospholipids may result in liposome compositions having advantageous activity or stability of activity properties. The above mentioned phospholipids may be mixed, in optimal ratios with cationic lipids, such as N-(1-(2,3-dioleolyoxy)propyl)-N,N,N-trimethyl ammonium chloride, 1,1'-dioctadecyl-3,3,3',3'-tetramethylindocarbocyanine perchloarate, 3,3'-deheptyloxacarbocyanine iodide, 1,1'-dedodecyl-3,3,3',3'-tetramethylindocarbocyanine perchloarate, 1,1'-dioleyl-3,3,3',3'-tetramethylindo carbocyanine methanesulfonate, N-4-(delinoleylaminostyryl)-N-methylpyridinium iodide, or 1,1,-dilinoleyl-3,3,3',3'-tetramethylindocarbocyanine perchloarate.

Liposomes may optionally comprise sphingolipids, in which spingosine is the structural counterpart of glycerol and one of the one fatty acids of a phosphoglyceride, or cholesterol, a major component of animal cell membranes. Liposomes may optionally contain pegylated lipids, which are lipids covalently linked to polymers of polyethylene glycol (PEG). PEGs may range in size from about 500 to about 10,000 daltons.

Liposomes may further comprise a suitable solvent. The solvent may be an organic solvent or an inorganic solvent. Suitable solvents include, but are not limited to, dimethylsulfoxide (DMSO), methylpyrrolidone, N-methylpyrrolidone, acetronitrile, alcohols, dimethylformamide, tetrahydrofuran, or combinations thereof.

Liposomes carrying the itaconate, malonate, derivatives thereof, an Adrenal corticotropin composition, and variant, or any combination thereof, may be prepared by any known method of preparing liposomes for drug delivery, such as, for example, detailed in U.S. Pat. Nos. 4,241,046; 4,394,448; 4,529,561; 4,755,388; 4,828,837; 4,925,661; 4,954,345; 4,957,735; 5,043,164; 5,064,655; 5,077,211; and 5,264,618, the disclosures of which are hereby incorporated by reference in their entirety. For example, liposomes may be prepared by sonicating lipids in an aqueous solution, solvent injection, lipid hydration, reverse evaporation, or freeze drying by repeated freezing and thawing. In a preferred embodiment the liposomes are formed by sonication. The liposomes may be multilamellar, which have many layers like an onion, or unilamellar. The liposomes may be large or small. Continued high-shear sonication tends to form smaller unilamellar lipsomes.

As would be apparent to one of ordinary skill, all of the parameters that govern liposome formation may be varied. These parameters include, but are not limited to, temperature, pH, concentration of an Adrenal corticotropin composition, variant, or any combination thereof, concentration and composition of lipid, concentration of multivalent cations, rate of mixing, presence of and concentration of solvent.

In another embodiment, a composition of the present disclosure may be delivered to a cell as a microemulsion. Microemulsions are generally clear, thermodynamically stable solutions comprising an aqueous solution, a surfactant, and "oil." The "oil" in this case, is the supercritical fluid phase. The surfactant rests at the oil-water interface. Any of a variety of surfactants are suitable for use in microemulsion formulations including those described herein or otherwise known in the art. The aqueous microdomains suitable for use in the present disclosure generally will have characteristic structural dimensions from about 5 nm to about 100 nm. Aggregates of this size are poor scatterers of visible light and hence, these solutions are optically clear. As will be appreciated by a skilled artisan, microemulsions can and will have a multitude of different microscopic structures including sphere, rod, or disc shaped aggregates. In one embodiment, the structure may be micelles, which are the simplest microemulsion structures that are generally spherical or cylindrical objects. Micelles are like drops of oil in water, and reverse micelles are like drops of water in oil. In an alternative embodiment, the microemulsion structure is the lamellae. It comprises consecutive layers of water and oil separated by layers of surfactant. The "oil" of microemulsions optimally comprises phospholipids. Any of the phospholipids detailed above for liposomes are suitable for embodiments directed to microemulsions. The compound of the itaconate, malonate, derivatives thereof, a compound of Formula (I), or a compound of Formula (II) may be encapsulated in a microemulsion by any method generally known in the art.

In yet another embodiment, an Adrenal corticotropin composition, prodrug, variant, or any combination thereof, may be delivered in a dendritic macromolecule, or a dendrimer. Generally speaking, a dendrimer is a branched tree-like molecule, in which each branch is an interlinked chain of molecules that divides into two new branches (molecules) after a certain length. This branching continues until the branches (molecules) become so densely packed that the canopy forms a globe. Generally, the properties of dendrimers are determined by the functional groups at their surface. For example, hydrophilic end groups, such as carboxyl groups, would typically make a water-soluble dendrimer. Alternatively, phospholipids may be incorporated in the surface of a dendrimer to facilitate absorption across the skin. Any of the phospholipids detailed for use in liposome embodiments are suitable for use in dendrimer embodiments. Any method generally known in the art may be utilized to make dendrimers and to encapsulate compositions of the present disclosure therein. For example, dendrimers may be produced by an iterative sequence of reaction steps, in which each additional iteration leads to a higher order dendrimer. Consequently, they have a regular, highly branched 3D structure, with nearly uniform size and shape. Furthermore, the final size of a dendrimer is typically controlled by the number of iterative steps used during synthesis. A variety of dendrimer sizes are suitable for use in the present disclosure. Generally, the size of dendrimers may range from about 1 nm to about 100 nm.

Generally, a safe and effective amount of an Adrenal corticotropin composition, prodrug, variant, or any combination thereof is, for example, that amount that would cause the desired therapeutic effect in a subject while minimizing undesired side effects.

The amount of a composition described herein that can be combined with a pharmaceutically acceptable carrier to produce a single dosage form will vary depending upon the host treated and the particular mode of administration. It will be appreciated by those skilled in the art that the unit content of agent contained in an individual dose of each dosage form need not in itself constitute a therapeutically effective amount, as the necessary therapeutically effective amount could be reached by administration of a number of individual doses.

Toxicity and therapeutic efficacy of compositions described herein can be determined by standard pharmaceutical procedures in cell cultures or experimental animals for determining the LD50 (the dose lethal to 50% of the population) and the ED50, (the dose therapeutically effective in 50% of the population). The dose ratio between toxic and therapeutic effects is the therapeutic index that can be expressed as the ratio LD50/ED50, where larger therapeutic indices are generally understood in the art to be optimal.

The specific therapeutically effective dose level for any particular subject will depend upon a variety of factors including the disorder being treated and the severity of the disorder; activity of the specific compound employed; the specific composition employed; the age, body weight, general health, sex and diet of the subject; the time of administration; the route of administration; the rate of excretion of the composition employed; the duration of the treatment; drugs used in combination or coincidental with the specific compound employed; and like factors well known in the medical arts (see e.g., Koda-Kimble et al. (2004) Applied Therapeutics: The Clinical Use of Drugs, Lippincott Williams & Wilkins, ISBN 0781748453; Winter (2003) Basic Clinical Pharmacokinetics, 4th ed., Lippincott Williams & Wilkins, ISBN 0781741475; Sharqel (2004) Applied Biopharmaceutics & Pharmacokinetics, McGraw-Hill/Appleton & Lange, ISBN 0071375503). For example, it is well within the skill of the art to start doses of the composition at levels lower than those required to achieve the desired therapeutic effect and to gradually increase the dosage until the desired effect is achieved. If desired, the effective daily dose may be divided into multiple doses for purposes of administration. Consequently, single dose compositions may contain such amounts or submultiples thereof to make up the daily dose. It will be understood, however, that the total daily usage of the compounds and compositions of the present disclosure will be decided by an attending physician within the scope of sound medical judgment.

Again, each of the states, diseases, disorders, and conditions, described herein, as well as others, can benefit from compositions and methods described herein. Generally, treating a state, disease, disorder, or condition includes preventing or delaying the appearance of clinical symptoms in a mammal that may be afflicted with or predisposed to the state, disease, disorder, or condition but does not yet experience or display clinical or subclinical symptoms thereof. Treating can also include inhibiting the state, disease, disorder, or condition, e.g., arresting or reducing the development of the disease or at least one clinical or subclinical symptom thereof. Furthermore, treating can include relieving the disease, e.g., causing regression of the state, disease, disorder, or condition or at least one of its clinical or subclinical symptoms. A benefit to a subject to be treated can be either statistically significant or at least perceptible to the subject or to a physician.

Administration of an adrenal corticotropin composition can occur as a single event or over a time course of treatment. For example, an adrenal corticotropin composition can be administered daily, weekly, bi-weekly, or monthly. For treatment of acute conditions, the time course of treatment will usually be at least several days. Certain conditions could extend treatment from several days to several weeks. For example, treatment could extend over one week, two weeks, or three weeks. For more chronic conditions, treatment could extend from several weeks to several months or even a year or more.

Treatment in accord with the methods described herein can be performed prior to, concurrent with, or after conventional treatment modalities. For instance, treatment in accord with the methods described herein can be performed prior to, concurrent with, or after conventional treatment modalities for an inflammatory autoimmune disease.

An adrenal corticotropin composition can be administered simultaneously or sequentially with another agent, such as an antibiotic, an anti-inflammatory, or other agent. For example, an adrenal corticotropin composition can be administered simultaneously with another agent, such as an anti-inflammatory agent. Simultaneous administration can occur through administration of separate compositions, each containing one or more of an adrenal corticotropin composition. Simultaneous administration can occur through administration of one composition containing two or more compositions. An adrenal corticotropin composition can be administered sequentially with an antibiotic, an anti-inflammatory, or another agent. For example, an adrenal corticotropin composition can be administered before or after administration of an anti-inflammatory, or another agent.

In some embodiments, one or more subsequent doses are administered every day, every other day, every two days, every three days, every four days, every 5 days, every 6 days, once a week, every two weeks, every three weeks, once a month, every six weeks, every two months, every three months, every four months, every five months, every six months or any combination thereof.

In some embodiments, a first dose of an adrenal corticotropin treatment is between about 10 IU, about 20 IU, about 30 IU, 40 IU, about 50 IU, about 60 IU, about 70 IU, 80 IU, about 90 IU, about 100 IU, about 110 IU, about 120 IU, about 130 IU, about 140 IU, about 150 IU about 200 IU, about 250 IU, about 300 IU, about 350 IU, about 400 IU, about 450 IU or about 500 IU, with reference to an ACTH peptide, fragment, or variant. In some embodiments, a first dose is between about 10 IU to about 400 IU, between about 10 IU to about 250 IU, between about 10 IU to about 100 IU, between about 10 IU to about 80 IU, between about 10 IU to about 60 IU, or between about 10 IU to about 40 IU. In some embodiments, a first dose is between about 10 IU to about 400 IU, between about 20 IU to about 400 IU, between about 40 IU to about 400 IU, between about 40 IU to about 350 IU, between about 40 IU to about 200 IU, between about 40 IU to about 100 IU, between about 40 IU to about 80 IU, or between about 40 IU to about 60 IU. In some embodiments, a first dose is between about 20 IU to about 200 IU, between about 60 IU to about 150 IU, between about 60 IU to about 100 IU, or between about 60 IU to about 80 IU.

In some embodiments, a one or more subsequent dose is between about 10 IU, about 20 IU, about 30 IU, 40 IU, about 50 IU, about 60 IU, about 70 IU, 80 IU, about 90 IU, about 100 IU, about 110 IU, about 120 IU, about 130 IU, about 140 IU, about 150 IU about 200 IU, about 250 IU, about 300 IU, about 350 IU, about 400 IU, about 450 IU or about 500 IU. In some embodiments, a one or more subsequent dose is between about 10 IU to about 400 IU, between about 10 IU to about 250 IU, between about 10 IU to about 100 IU, between about 10 IU to about 80 IU, between about 10 IU to about 60 IU, or between about 10 IU to about 40 IU. In some embodiments, a one or more subsequent dose is between about 10 IU to about 400 IU, between about 20 IU to about 400 IU, between about 40 IU to about 400 IU, between about 40 IU to about 350 IU, between about 40 IU to about 200 IU, between about 40 IU to about 100 IU, between about 40 IU to about 80 IU, or between about 40 IU to about 60 IU. In some embodiments, a one or more subsequent dose is between about 20 IU to about 200 IU, between about 60 IU to about 150 IU, between about 60 IU to about 100 IU, or between about 60 IU to about 80 IU.

In some embodiments, the pharmaceutical compositions described herein are in unit dosage forms suitable for single administration of precise dosages. In unit dosage form, the formulation is divided into unit doses containing appropriate quantities of an adrenal corticotropin treatment. In some embodiments, the unit dosage is in the form of a package containing discrete quantities of a formulation. Non-limiting examples are packaged tablets or capsules, powders in vials or ampoules, or injectable suspension or solution in ampoules. In some embodiments, aqueous suspension compositions are packaged in single-dose non-reclosable containers. Alternatively, multiple-dose reclosable containers are used. In some of such embodiments, a preservative is optionally included in the composition. By way of example only, formulations for intramuscular injection are presented in unit dosage form, which include, but are not limited to ampoules, or in multi dose containers, with an added preservative. In particular embodiments, Acthar® Gel may be administered via a self-injector device.

### (e) combinations

Compositions disclosed herein may be administered in combination with one or more other therapeutic agents, such as chemotherapy drugs, radiation, monoclonal antibodies, immune cells such as T cells, immunosuppressive agents including cyclosporine, tacrolimus, CTLA-4Ig, TNF inhibitors, IL-1 receptor blockers, anti-CD20 antibodies, inhibitors of IL-6, inhibitors of IL-17, inhibitors of IL-23, immune checkpoint drugs, cyclophosphamide, methotrexate, azathioprine, glucocorticoids, anabolic steroids, anti-inflammatory agents including non-steroidal anti-inflammatory drugs, aspirin, colchicine, indomethacin, phenylbutazone, drugs that decrease plasma uric acid levels, volume repleting agents, anti-epileptics, drugs to treat diabetes, drugs to treat hypovolemic shock, drugs to treat multiple sclerosis, drugs to treat kidney disease, drugs to treat asthma, drugs to treat gout, drugs to treat arthritis, drugs to treat immunodeficiencies, drugs to treat GvHD, and drugs to treat osteoporosis. Such combination administration may be by means of a single dosage form which includes both a composition disclosed herein and one more other pharmaceutically active compounds, for example in a tablet, capsule, spray, inhalation powder, injectable liquid or the like. Alternatively, combination administration may be by means of administration of two different dosage forms, with one dosage form containing a composition disclosed herein, and the other dosage form including another pharmaceutically active compound. In this instance, the dosage forms may be the same or different. The term "co-administer" indicates that each of at least two compounds in the combination therapy are administered during a time frame wherein the respective periods of biological activity or effects overlap. Thus the term includes sequential las well as concurrent administration. In some embodiments, compositions disclosed herein can be chemically linked to other therapeutic agents prior to or for the purpose of co-administration. For example, compositions can be linked to, e.g., a monoclonal antibody or other protein, such as CTLA-4 Ig or etanercept. If more than one compound is co-administered, the routes of administration of the two or more compounds need not be the same.

### II. METHODS

The present disclosure encompasses methods of choosing an adrenal corticotropin treatment, methods of designing a corticosteroid response, and methods of treatment. Each is described in more detail below.

In each of the below embodiments, the phrase "a subject in need thereof" refers to a subject in need of preventative or therapeutic treatment. A subject may be a human, a rodent, a livestock animal, a companion animal, or a zoological animal. In one embodiment, a subject may be a rodent, *e.g.*, a mouse, a rat, a guinea pig, etc. In another embodiment, a subject may be a livestock animal. Non-limiting examples of suitable livestock animals may include pigs, cows, horses, goats, sheep, llamas and alpacas. In still another embodiment, a subject may be a companion animal. Non-limiting examples of companion animals may include pets such as dogs, cats, rabbits, and birds. In yet another embodiment, a subject may be a zoological animal. As used herein, a "zoological animal" refers to an animal that may be found in a zoo. Such animals may include non-human primates, large cats, wolves, and bears. In another preferred embodiment, a subject is a human.

In each of the embodiments detailed below, a subject in need of treatment or in need of a designed cortiocosteroid response may be a subject diagnosed with one or more inflammatory diseases, disorders, or conditions. An inflammatory condition may result from any disease or disorder, such as, but not limited to, solar urticaria, xeroderma pigmentosa, diabetic retinopathy, retinopathy of prematurity, uveitis, gingivitis, mucositis, peritonitis, pleuritis, endometriosis, peripheral vascular disease, arthritic conditions such as osteoarthritis, juvenile rheumatoid arthritis, rheumatoid arthritis, septic arthritis, psoriatic arthritis, acute gout, gout and pseudogout, juvenile idiopathic arthritis, Still's disease and ankylosing spondylitis, as well as arthritis secondary to other diseases, such as arthritis secondary to lupus erythematosus, Henoch-Schonlein purpura, psoriasis, reactive arthritis, haemochromatosis, hepatitis including toxic hepatitis, Wegener's granulomatosis, vasculitis syndromes, Lyme disease, familial Mediterranean fever, hyperimmunoglobulinemia D with recurrent fever, TNF receptor-associated periodic syndrome and inflammatory bowel disease, including Crohn's disease and ulcerative colitis. In addition, inflammation conditions can result from joint damage due to sickle cell anemia and bleeding into the joint associated with hemophilia.

An inflammatory condition may result from inflammatory bowel disease, such as Crohn's disease, eosinophilic esophagitis, ulcerative colitis, collagenous colitis, lymphocytic colitis, ischemic colitis, diversion colitis, Behcet's syndrome, infective colitis and indeterminate colitis.

An inflammatory condition may result from an autoimmune disease, including, but not limited to, systemic syndromes such as systemic lupus erythematosus, Sjogren's syndrome, scleroderma, juvenile rheumatoid arthritis, rheumatoid arthritis, psoriatic arthritis, polymyositis, and dermatomyositis. An autoimmune disease may also be a local condition affecting biological subsystems such as the endocrine system (e.g., diabetes mellitus type 1, Hashimoto's thyroiditis, and Addison's disease); the dermatologic system (e.g., pemphigus vulgaris); the hematologic system (e.g., autoimmune hemolytic anemia); the renal system(e.g., nephrotic syndrome and nephritis such as glomerulonephritis and interstitial nephritis); and the neural system (e.g., multiple sclerosis). Additional, non-limiting examples of autoimmune diseases and autoimmune disease related disorders include acute disseminated encephalomyelitis, ankylosing spondylitis, antiphospholipid antibody syndrome, aplastic anemia, autoimmune hepatitis, toxic hepatitis, autoimmune oophoritis, autoimmune thrombocytopenia, autoimmune hemolyticanemia, drug-induced antibodies, drug reactions from administration of biologics, celiac disease, Crohn's disease, gestational pemphigoid, Good pasture's syndrome, Graves' disease, Guillain-Barre syndrome, Hashimoto's disease, idiopathic thrombocytopenic purpura, irritable bowel disease (IBD), Kawasaki disease, lupus erythematosus, mixed connective tissue disease, multiple sclerosis, myasthenia gravis, opsoclonus myoclonus syndrome, optic neuritis, Ord's thyroiditis, pemphigus, pernicious anaemia, primary biliary cirrhosis, Reiter's syndrome, Sjogren's syndrome, Takayasu's arteritis, temporal arteritis, autoimmunehemolytic anemia, Wegener's granulomatosis, spasms, infantile spasms, and epilepsy.

An inflammatory condition may result from a skin condition, such as contact dermatitis, atopic dermatitis, Stevens-Johnson syndrome, burns, and acne.

An inflammatory condition may result from pancreatitis, which can be caused by gallstones, mumps, tumors, pain medication, antibiotics, or from alcohol use.

An inflammatory condition may result from vasculitis, Behcet's Disease, Buerger's disease (thromboangiitis obliterans), eosinophilic granulomatosis with polyangiitis (EGPA or Churg Strauss Syndrome), cryoglobulinemia, giant cell arteritis (or temporal arteritis), Henoch-Schonlein purpura (or IgA vasculitis), microscopic polyangiitis, polyarteritis nodosa, polymyalgia rheumatic, rheumatoid vasculitis, Takayasu's arteritis, hypersensitivity vasculitis, Kawasaki disease, isolated aortitis, urticarial vasculitis, retinal vasculitis, and granulomatosis with polyangiitis (GPA or Wegener's granulomatosis).

The inflammation can be associated with an acute inflammatory condition. Non-limiting examples of acute inflammatory conditions include acute flares of inflammatory arthritis, including RA, lupus and other autoimmune diseases, MS flares; acute attacks of gout; acute attacks of asthma; severe acute allergic reactions such as anaphylaxis and shock; acute injuries to tissues such as the skin (e.g., burns, dermatitis, and phototoxic skin reactions), brain (e.g., stroke) or heart (e.g., myocardial infarction), liver, kidney, lungs, and pancreas (e.g., pancreatitis), gastrointestinal tract including esophagus, stomach, intestine (small and large), rectum, all of which could be due to toxic injury, ischemia/infarction, inflammation, or trauma; shock due to hypovolemia, sepsis, or toxic shock syndrome; acute respiratory distress syndrome; vaccine reaction; and cytokine storm secondary to immunotherapy for cancer or other causes.

An inflammatory reaction may result from an immune response to a foreign molecule or cell introduced into the body. This could be a drug such as an anti-hemophilic factor being administered to treat a medical condition. These include proteins, monoclonal antibodies, peptides, polypeptides, and cells. Examples include reactions to monoclonal antibodies, such as rituximab; anti-hemophilic factors, such as FactorsVII, VIII, IX, and X; and other proteins, such as interferons including alpha, beta, and gamma. An inflammatory reaction may also be in response to a vaccine.

An inflammatory reaction may result from a neurological disorder characterized by myelin loss or myelin deficiency. Non-limiting examples of disorders characterized by myelin loss or myelin deficiency include, for example, multiple sclerosis (MS) (e.g., Relapsing/Remitting Multiple Sclerosis, Secondary Progressive Multiple Sclerosis, Progressive Relapsing Multiple Sclerosis, Primary Progressive Multiple Sclerosis, and Acute Fulminant Multiple Sclerosis), Central Pontine Myelinolysis, Acute Disseminated Encephalomyelitis, Progressive Multifocal Leukoencephalopathy, Subacute S clerosing Panencephalitis, Post-infectious Encephalomyelitis, Chronic Inflammatory Demyelinating Polyneuropathy, Devic's Disease, Balo's Concentric Sclerosis, the leukodystrophies (e.g., Metachromatic Leukodystrophy, Krabbe disease, Adrenoleukodystrophy, Pelizaeus-Merzbacher disease, Canavan disease, Childhood Ataxia with Central Hypomyelination, Alexander disease, or Refsum disease), optic neuritis, transverse myelitis, cerebral palsy, spinal cord injury, age-associated myelin deficiency, as well as acquired and inherited neuropathies in the peripheral nervous system (e.g., Guillain-Barre Syndrome and Charcot Marie Tooth disease).

An inflammatory condition may result from a chronic obstructive pulmonary disease (COPD), also known as chronic obstructive airway diseases. These can include diseases characterized by the pathological limitation of airflow in the airway that is not fully reversible, for example chronic bronchitis, emphysema, pneumoconiosis, pulmonary neoplasms and other lung disorders. Other inflammatory conditions may include upper or lower airway diseases and disorders, such as allergic asthma, eosinophilic pneumonia, alpha-1 anti-trypsin deficiency, allergic rhinitis, vasomotor rhinitis, allergic conjunctivitis, non-allergic conjunctivitis, Wegener's granulomatosis, and sarcoidosis; and airway diseases related to external toxins or substances including various forms of pneumoconiosis (coal worker's pneumoconiosis, asbestosis, silicosis, bauxite fibrosis, berylliosis, or siderosis), byssinosis or hypersensitivity pneumonitis (farmer's lung or bird fancier's lung). Other lung diseases involving an inflammatory condition include acute respiratory distress syndrome.

The inflammatory condition may result from a transplant-related condition or syndrome, such as graft-versus-host disease, hyper-acute rejection, acute rejection, or chronic rejection. Graft-versus-host disease (GVHD) is a common complication of allogeneic bone marrow transplantation, but can occur with other transplantations, particularly those with T-cells present in the graft, either as contaminants or intentionally introduced. GVHD may be characterized as acute or chronic. Hyper-acute, acute or chronic rejection can occur with bodily organs such as kidneys, liver, pancreas, spleen, uterus, heart or lungs, as well as transplantation of bone, cornea, face, hand, penis or skin.

The inflammatory condition may result from a muscular dystrophy, which refers to diseases associated with muscle and the musculoskeletal system. Inflammation can be associated with muscular dystrophies such as Duchenne Muscular Dystrophy and Becker's Muscular Dystrophy. An inflammatory condition may result from sarcopenia, for example sarcopenia due to aging or chronic disease, e.g., cachexia. Inflammation may result from muscle injury.

The inflammatory condition may result from cystic fibrosis, which is a hereditary disease associated with a mutation in the gene cystic fibrosis transmembrane conductance regulator.

The inflammatory condition may result from a nervous system condition, such as a central nervous system condition. Non-limiting examples of inflammatory conditions affecting the nervous system include the various types of multiple sclerosis (e.g., relapse-remitting, secondary progressive, primary progressive, and progressive-relapsing);various types of Alzheimer's disease; Parkinson's disease; spinal muscular atrophy; various types of encephalitis and meningitis (e.g., primary encephalitis, secondary encephalitis, bacterial meningitis, viral meningitis, parasitic meningitis, fungal meningitis, and non-infectious meningitis); acute disseminated encephalomyelitis; acute transverse myelitis; neuro myelitis optica; focal demyelinating syndromes (e.g.,Balo's concentric sclerosis and Marburg variant of MS); amyotrophiclateral sclerosis; progressive multifocal leukoencephalopathy; subacutesclerosing panencephalitis; acute haemorrhagic leucoencephalitis (Hurst's disease); human T-lymphotropic virus type-1-associatedmyelopathy/tropical spactic paraparesis; Devic's disease; human immunodeficiency virus encephalopathy; human immunodeficiency virus vacuolar myelopathy; peripheral neuropathies; Guillanin-Barre Syndrome and other immune mediated neuropathies; and myasthenia gravis.

A neural inflammatory condition may be associated with a cerebrovascular accident or a peripheral nerve disorder, such as peripheral neuropathy, autonomic neuropathy, mononeuropathy, sciatica, carpal tunnel syndrome, polyneuropathy, diabetic neuropathy, postherpetic neuralgia, and thoracic outlet syndrome.

The inflammatory condition may result from trauma, such as injury to tissues and organs, including bone, muscle, heart, kidney, spleen, lung, liver, brain, thyroid, bladder, gall bladder, diaphragm, stomach, pancreas, intestine, breast, and rectum.

The inflammatory condition may result from adhesions, including visceral, peritoneal, and pleural, from surgery, trauma or other injuries as well as inflammatory disorders.

The inflammatory condition may result from a skeletal disorder, which comprises a group of heterogeneous diseases affecting cells and tissues of bone, bone marrow, cartilage, tendons, and ligaments. A skeletal disorder may include genetic disorders characterized by abnormal bone metabolism or resorption. Non-limiting examples of skeletal disorders and conditions include osteoporosis (e.g., postmenopausal osteoporosis, glucocorticoid induced osteoporosis, hyperthyroidism-induced osteoporosis, immobilization-induced osteoporosis, heparin-induced osteoporosis and immunosuppressive-induced osteoporosis as well as long term complications of osteoporosis such as curvature of the spine, loss of height and prosthetic surgery), abnormally increased bone turnover, hyper-calcemia including humora Ihypercalcemia, hyperparathyroidism, Paget's bone diseases, osteolysis including periprosthetic osteolysis, hypercalcemia of malignancy with or without bone metastases, hypercalcemia of fracture healing, juvenile rheumatoid arthritis, rheumatoid arthritis, osteoarthritis, ostealgia, osteopenia, calculosis, lithiasis including urolithiasis, gout and ankylosing spondylitis, tendonitis, bursitis, malignant bone tumor e.g. osteosarcoma, osteogenesis imperfecta, metastatic bone disease, alveolar bone loss, post-osteomy and childhood idiopathic bone loss.

The inflammatory condition may result from a spinal condition, such as cervical disk rupture, degenerative disc disease, lumbar disc disease, compression fracture, spinal stenosis, myelopathy, lumbar scoliosis, kyphosis, spondylolisthesis, anklylosing spondylitis, and retrolisthesis.

An inflammatory condition may result from immunodeficiencies, including common variable immunodeficiency, selective immunoglobulin deficiency, IgA deficiency, transient hypogammaglobulinemia of infancy, X-linked agammaglobulinemia, chronic mucocutaneous candidiasis, DiGeorge syndrome, X-linked lymphoproliferative syndrome, Ataxiatelangiectasia, hyperimmunoglobulinemia E syndrome, severe combined immunodeficiency, Wiskott-Aldrich syndrome, chronic granulomatous disease, Chediak-Higashi syndrome, cyclic neutropenia, leuckocyte adhesion defects, complement component 1 (C1) inhibitor deficiency (hereditary angioedema), complement component 3 (C3) deficiency, complement component 4 (C4) deficiency, complement component 5 (C5) deficiency, complement component 6 (C6)deficiency, complement component 7 (C7) deficiency, complement component 8 (C8) deficiency, or complement component 9 (C9) deficiency.

An inflammatory condition may result from immunodeficiencies that are iatrogenic, such as treatment with corticosteroids, immunosuppressive drugs such as cyclosporine, methotrexate, cytotoxic agents such as cyclophosphamide, and radiation.

### *(a) methods of choosing an adrenal corticotropin treatment*

One aspect of the present disclosure encompasses a method of choosing an adrenal corticotropin treatment for a subject in need of such treatment. The method generally comprises (a) determining if a delayed corticosteroid response or a non-delayed response is appropriate for the subject; and (b) administering an adrenal corticotropin treatment consisting essentially of an RCI dose if a non-delayed response is appropriate or administering an adrenal corticotropin treatment consisting essentially of a synthetic adrenal corticotropin composition if a delayed response is appropriate.

Another aspect of the present disclosure encompasses a method of choosing an adrenal corticotropin treatment for a subject in need of such treatment, where the method generally comprises (a) determining if a high daily steroidal equivalent dose response or a low daily steroidal equivalent dose response is appropriate for the subject; and (b) administering an adrenal corticotropin treatment consisting essentially of an RCI dose if a low daily steroidal equivalent dose response is appropriate or administering an adrenal corticotropin treatment consisting essentially of a synthetic adrenal corticotropin composition if a high daily steroidal equivalent dose response is appropriate.

A corticosteroid response refers to a rise in a subject's blood corticosteroid level. A corticosteroid response may comprise a rise in one or more of the following corticosteroids: 11-Dehydrocorticosterone (11-oxocorticosterone; 17-deoxycortisone; 21-hydroxypregn-4-ene-3,11,20-trione); 11-Deoxycorticosterone (deoxycortone, desoxycortone; 21-hydroxyprogesterone; 21-hydroxypregn-4-ene-3,20-dione); 11-Deoxycortisol (cortodoxone, cortexolone; $17\alpha$,21-dihydroxypregn-4-ene-3,20-dione); 11-Ketoprogesterone (11-oxoprogesterone; Ketogestin; pregn-4-ene-3,11,20-trione); $11\beta$-Hydroxypregnenolone ($3\beta$,$11\beta$-dihydroxypregn-5-en-20-one); $11\beta$-Hydroxyprogesterone (21-deoxycorticosterone; $11\beta$-hydroxypregn-4-ene-3,20-dione); $11\beta$,$17\alpha$,21-Trihydroxypregnenolone ($3\beta$,$11\beta$,$17\alpha$,21-tetrahydroxypregn-5-en-20-one); $17\alpha$,21-Dihydroxypregnenolone ($3\beta$,$17\alpha$,21-trihydroxypregn-5-en-20-one); $17\alpha$-Hydroxypregnenolone ($3\beta$,$17\alpha$-dihydroxypregn-5-en-20-one); $17\alpha$-Hydroxyprogesterone ($17\alpha$-hydroxypregn-4-ene-3,11,20-trione); 18-Hydroxy-11-deoxycorticosterone (18,21-dihydroxypregn-4-ene-3,20-dione); 18-Hydroxycorticosterone ($11\beta$,18,21-trihydroxypregn-4-ene-3,20-dione); 18-Hydroxyprogesterone (18-hydroxypregn-4-ene-3,20-dione); 21-deoxycortisol ($11\beta$,$17\alpha$-dihydroxypregn-4-ene-3,20-dione); 21-Deoxycortisone ($17\alpha$-hydroxypregn-4-ene-3,11,20-trione); 21-Hydroxypregnenolone (prebediolone; $3\beta$,21-dihydroxypregn-5-en-20-one); aldosterone ($11\beta$,21-dihydroxypregn-4-ene-3,18,20-trione); corticosterone (17-deoxycortisol; $11\beta$,21-dihydroxypregn-4-ene-3,20-dione); cortisol (hydrocortisone; $11\beta$,$17\alpha$,21-trihydroxypregn-4-ene-3,20-dione); cortisone ($17\alpha$,21-dihydroxypregn-4-ene-3,11,20-trione); pregnenolone (pregn-5-en-$3\beta$-ol-20-one); progesterone (pregn-4-ene-3,20-dione).

In some embodiments, a corticosteroid response may be a rise in a subject's blood glucocorticoid (glucocorticoidsteroid) level, e.g. a glucocorticoid response. A glucocorticoid is a corticosteroid that binds to the glucocorticoid receptor. A glucocorticoid response may comprise a rise in blood level of corticosterone or cortisol. In particular embodiments, a corticosteroid response may be a rise in cortisol, e.g. a cortisol response.

As used herein, "delayed corticosteroid response" refers to a response where a subject's peak corticosteroid blood level occurs at a time point greater than 8 hours after the administration of the adrenal corticotropin treatment. For instance, in some embodiments, a delayed corticosteroid response in a subject will have a peak corticosteroid blood level at least 8, 9, 10, 11, 12 or more than 12 hours after administration of the adrenal corticotropin treatment. In preferred embodiments, a delayed corticosteroid response in a subject will have a peak corticosteroid blood level at least 9 or more hours after administration of the adrenal corticotropin treatment.

As used herein, the phrase "non-delayed corticosteroid response" refers to a response where a subject's peak corticosteroid blood level occurs at a time point less than 8 hours after the administration of the adrenal corticotropin treatment. For instance, in some embodiments, a non-delayed corticosteroid response in a subject will have a peak corticosteroid blood level at 0.5, 1, 2, 3, 4, 5, 6, 7, or less than 8 hours after the administration of the adrenal corticotropin treatment.

As used herein, a "low daily steroidal equivalent dose" refers to a dose of adrenal corticotropin treatment that induces a daily steroidal equivalent dose of less than or equal to 20 mg/d of prednisone. Methods of calculating a daily steroidal equivalent dose are detailed in Example 2 below. In some embodiments, a low daily steroidal equivalent dose refers to a dose of less than or equal to 20, 19, 18, 17, 16, 15, 14, 13, 12, 11, 10, 9, 8, 7, 6, 5, 4, 3, 2, or 1 mg/d of prednisone. In particular embodiments, a low daily steroidal equivalent dose refers to a dose of less than or equal to 10 mg/d prednisone.

As used herein, a "high daily steroidal equivalent dose" refers to a dose of adrenal corticotropin treatment that induces a daily steroidal equivalent dose of greater than 20 mg/d of prednisone. As stated above, methods of calculating a daily steroidal equivalent dose are detailed in Example 2 below. In some embodiments, a high daily steroidal equivalent dose refers to a dose of greater than 20, 30, 40, 50, 60, 70, or 80 mg/d of prednisone.

Determining if a delayed corticosteroid response or a non-delayed response is appropriate for the subject is a determination that a medical professional, with the ordinary level of skill expected of such a professional, can readily determine. Such a professional may consider the constitution of the subject, the disease, disorder, or condition that the subject has been diagnosed with or is at risk for, the previous treatments administered to the patient, and the current medications taken by the subject.

Similarly, determining if a low daily steroidal equivalent dose or a high daily steroidal equivalent dose is appropriate for the subject is a determination that a medical professional, with the ordinary level of skill expected of such a professional, can readily determine. Such a professional may consider the constitution of the subject, the disease, disorder, or condition that the subject has been diagnosed with or is at risk for, the previous treatments administered to the patient, and the current medications taken by the subject. In particular embodiments, a low daily steroidal equivalent dose may be appropriate for a subject that has shown a poor clinical response to corticosteroids. As used herein, "poor clinical response" refers to subjects that have shown no improvement or a worsening of clinical symptoms after administration of corticosteroids. In other embodiments, a low daily steroidal equivalent dose may be appropriate for subjects that have become resistant to the effects of corticosteroids. As used herein, the phrase "resistant to the effects of corticosteroids" refers to a subject that initially showed a positive clinical response to a corticosteroid, but after repeated dosing, is now showing a poor clinical response to a corticosteroid. In still other embodiments, a low daily steroidal equivalent dose may be appropriate for subjects who are unable to tolerate the side effects of corticosteroids. A determination that a subject is unable to tolerate the side effects of corticosteroids is a determination a medical professional, with the ordinary level of skill expected of such a professional, can readily make.

A subject in need of adrenal corticotropin treatment may be a subject diagnosed with an autoimmune disorder.

### (b) methods of designing a corticosteroid response in a subject

Another aspect of the present disclosure comprises a method of designing a corticosteroid response in a subject. The method comprises: (a) selecting a subject in need of a designed corticosteroid response, (b) designing an appropriate corticosteroid response for the subject, and (c) administering one or more doses of an adrenal corticotropin treatment to the subject at a dose sufficient to invoke the desired corticosteroid response.

A corticosteroid response refers to a rise in a subject's blood corticosteroid level. A corticosteroid response may comprise a rise in one or more of the following corticosteroids: 11-Dehydrocorticosterone (11-oxocorticosterone; 17-deoxycortisone; 21-hydroxypregn-4-ene-3,11,20-trione); 11-Deoxycorticosterone (deoxycortone, desoxycortone; 21-hydroxyprogesterone; 21-hydroxypregn-4-ene-3,20-dione); 11-Deoxycortisol (cortodoxone, cortexolone; $17\alpha$,21-dihydroxypregn-4-ene-3,20-dione); 11-Ketoprogesterone (11-oxoprogesterone; Ketogestin; pregn-4-ene-3,11,20-trione); $11\beta$-Hydroxypregnenolone ($3\beta$,$11\beta$-dihydroxypregn-5-en-20-one); $11\beta$-Hydroxyprogesterone (21-deoxycorticosterone; $11\beta$-hydroxypregn-4-ene-3,20-dione); $11\beta$,$17\alpha$,21-Trihydroxypregnenolone ($3\beta$,$11\beta$,$17\alpha$,21-tetrahydroxypregn-5-en-20-one); $17\alpha$,21-Dihydroxypregnenolone ($3\beta$,$17\alpha$,21-trihydroxypregn-5-en-20-one); $17\alpha$-Hydroxypregnenolone

(3β,17α-dihydroxypregn-5-en-20-one); 17α-Hydroxyprogesterone (17α-hydroxypregn-4-ene-3,11,20-trione); 18-Hydroxy-11-deoxycorticosterone (18,21-dihydroxypregn-4-ene-3,20-dione); 18-Hydroxycorticosterone (11β,18,21-trihydroxypregn-4-ene-3,20-dione); 18-Hydroxyprogesterone (18-hydroxypregn-4-ene-3,20-dione); 21-deoxycortisol (11β,17α-dihydroxypregn-4-ene-3,20-dione); 21-Deoxycortisone (17α-hydroxypregn-4-ene-3,11,20-trione); 21-Hydroxypregnenolone (prebediolone; 3β,21-dihydroxypregn-5-en-20-one); aldosterone (11β,21-dihydroxypregn-4-ene-3,18,20-trione); corticosterone (17-deoxycortisol; 11β,21-dihydroxypregn-4-ene-3,20-dione); cortisol (hydrocortisone; 11β,17α,21-trihydroxypregn-4-ene-3,20-dione); cortisone (17α,21-dihydroxypregn-4-ene-3,11,20-trione); pregnenolone (pregn-5-en-3β-ol-20-one); progesterone (pregn-4-ene-3,20-dione).

In some embodiments, a corticosteroid response may be a rise in a subject's blood glucocorticoid (glucocorticoidsteroid) level, e.g. a glucocorticoid response. A glucocorticoid is a corticosteroid that binds to the glucocorticoid receptor. A glucocorticoid response may comprise a rise in blood level of corticosterone or cortisol. In particular embodiments, a corticosteroid response may be a rise in cortisol, e.g. a cortisol response.

In some embodiments, a corticosteroid response may be designed to be a delayed corticosteroid response, as defined in section II(a) above. For instance, in some embodiments, a delayed corticosteroid response in a subject will have a peak corticosteroid blood level at least 8, 9, 10, 11, 12 or more than 12 hours after administration of the adrenal corticotropin treatment. In embodiments where a delayed corticosteroid response is needed or desired, the subject may be administered an adrenal corticotropin treatment that consists essentially of a synthetic adrenal corticotropin composition, as described in section I above.

In other embodiments, a corticosteroid response may be designed to be a non-delayed response, as defined in section II(a) above. For instance, in some embodiments, a non-delayed corticosteroid response in a subject will have a peak corticosteroid blood level at 0.5, 1, 2, 3, 4, 5, 6, 7, or less than 8 hours after the administration of the adrenal corticotropin treatment. In embodiments where a non-delayed corticosteroid response is needed or desired, the subject may be administered an adrenal corticotropin treatment that consists essentially of a non-synthetic adrenal corticotropin composition. In preferred embodiments, such a non-synthetic composition is an RCI composition as detailed in section I above. In a particularly preferred embodiment, such an RCI composition is Acthar® Gel.

In still other embodiments, a corticosteroid response may be designed to be a low daily steroidal equivalent dose response, as defined in section II(a) above. For instance, in some embodiments, a low daily steroidal equivalent dose response in a subject will have a daily steroidal equivalent dose of less than or equal to 20 mg/d of prednisone. In some embodiments, a low daily steroidal equivalent dose refers to a dose of less than or equal to 20, 19, 18, 17, 16, 15, 14, 13, 12, 11, 10, 9, 8, 7, 6, 5, 4, 3, 2, or 1 mg/d of prednisone. In particular embodiments, a low daily steroidal equivalent dose refers to a dose of less than or equal to 10 mg/d prednisone. In embodiments where a low daily steroidal equivalent dose response is needed or desired, the subject may be administered an adrenal corticotropin treatment that consists essentially of a non-synthetic adrenal corticotropin composition. In preferred embodiments, such a non-synthetic composition is an RCI composition as detailed in section I above. In a particularly preferred embodiment, such an RCI composition is Acthar® Gel.

In still further embodiments, a corticosteroid response may be designed to be a high daily steroidal equivalent dose response, as defined in section II(a) above. For instance, in some embodiments, a high daily steroidal equivalent dose response refers to a dose of adrenal corticotropin treatment that induces a daily steroidal equivalent dose of greater than 20 mg/d of prednisone. In some embodiments, a high daily steroidal equivalent dose refers to a dose of greater than 20, 30, 40, 50, 60, 70, or 80 mg/d of prednisone. In embodiments where a high daily steroidal equivalent response dose is needed or desired, the subject may be administered an adrenal corticotropin treatment that consists essentially of a synthetic adrenal corticotropin composition, as described in section I above.

In particular embodiments, a corticosteroid response may be designed to be a delayed, high daily steroidal equivalent dose response. In such embodiments, the subject may be administered an adrenal corticotropin treatment that consists essentially of a synthetic adrenal corticotropin composition, as described in section I above.

In other particular embodiments, a corticosteroid response may be designed to be a non-delayed, low daily steroidal equivalent dose response. In such embodiments, the subject may be administered an adrenal corticotropin treatment that consists essentially of a non-synthetic adrenal corticotropin composition. In preferred embodiments, such a non-synthetic composition is an RCI composition as detailed in section I above. In a particularly preferred embodiment, such an RCI composition is Acthar® Gel.

In particular embodiments, a corticosteroid response may be designed to be dose-dependent. That is, the subject's corticosteroid response may be increased or decreased depending on the dosage of adrenal corticotropin treatment administered to the subject. When such dose dependency is needed or desired, the subject may be administered an adrenal corticotropin treatment that consists essentially of a non-synthetic adrenal corticotropin composition. In preferred embodiments, such a non-synthetic composition is an RCI composition as detailed in section I above. In a particularly preferred embodiment, such an RCI composition is Acthar® gel.

In certain embodiments, a corticosteroid response may be non-dose dependent. That is, the subject's corticosteroid response will not substantially change based on the dosage of adrenal corticotropin treatment administered to the subject. In embodiments where a non-dose dependent corticosteroid response is needed or desired, the subject may be administered an adrenal corticotropin treatment that consists essentially of a synthetic adrenal corticotropin composition, as described in section I above.

Determining the characteristics of the desired corticosteroid response that is appropriate for the subject is a determination that a medical professional, with the ordinary level of skill expected of such a professional, can determine. Such a professional may consider the constitution of the subject, the disease, disorder, or condition that the subject has been diagnosed with or is at risk for, the previous treatments administered to the patient, and the current medications taken by the subject. In particular embodiments, a low daily steroidal equivalent dose may be appropriate for a subject that has shown a poor clinical response to corticosteroids. In other embodiments, a low daily steroidal equivalent dose may be appropriate for subjects that have become resistant to the effects of corticosteroids. In still other embodiments, a low daily steroidal equivalent dose may be appropriate for subjects who are unable to tolerate the side effects of corticosteroids. A determination that a subject is unable to tolerate the side effects of corticosteroids is a determination a medical professional, with the ordinary level of skill expected of such a professional, can readily make.

### (c) methods of treatment

Yet another aspect of the present disclosure encompasses a method of treating a subject. The method comprises: (a) determining if a strong corticosteroid response in the subject is beneficial; (b) when the answer to step (a) is no, administering one or more doses of repository corticotropin injection (RCI) to the subject at a dose sufficient to not invoke a strong corticosteroid response in the subject, wherein the desired corticosteroid response aids in the treatment of the subject. Alternatively, if the answer is yes, the subject may be administered an adrenal corticotropin treatment that consists essentially of a synthetic adrenal corticotropin composition, as described in section I above.

A corticosteroid response refers to a rise in a subject's blood corticosteroid level. A corticosteroid response may comprise a rise in one or more of the following corticosteroids: 11-Dehydrocorticosterone (11-oxocorticosterone; 17-deoxycortisone; 21-hydroxypregn-4-ene-3,11,20-trione); 11-Deoxycorticosterone (deoxycortone, desoxycortone; 21-hydroxyprogesterone; 21-hydroxypregn-4-ene-3,20-dione); 11-Deoxycortisol (cortodoxone, cortexolone; $17\alpha$,21-dihydroxypregn-4-ene-3,20-dione); 11-Ketoprogesterone (11-oxoprogesterone; Ketogestin; pregn-4-ene-3,11,20-trione); $11\beta$-Hydroxypregnenolone ($3\beta$,$11\beta$-dihydroxypregn-5-en-20-one); $11\beta$-Hydroxyprogesterone (21-deoxycorticosterone; $11\beta$-hydroxypregn-4-ene-3,20-dione); $11\beta$,$17\alpha$,21-Trihydroxypregnenolone ($3\beta$,$11\beta$,$17\alpha$,21-tetrahydroxypregn-5-en-20-one); $17\alpha$,21-Dihydroxypregnenolone ($3\beta$,$17\alpha$,21-trihydroxypregn-5-en-20-one); $17\alpha$-Hydroxypregnenolone ($3\beta$,$17\alpha$-dihydroxypregn-5-en-20-one); $17\alpha$-Hydroxyprogesterone ($17\alpha$-hydroxypregn-4-ene-3,11,20-trione); 18-Hydroxy-11-deoxycorticosterone (18,21-dihydroxypregn-4-ene-3,20-dione); 18-Hydroxycorticosterone ($11\beta$,18,21-trihydroxypregn-4-ene-3,20-dione); 18-Hydroxyprogesterone (18-hydroxypregn-4-ene-3,20-dione); 21-deoxycortisol ($11\beta$,$17\alpha$-dihydroxypregn-4-ene-3,20-dione); 21-Deoxycortisone ($17\alpha$-hydroxypregn-4-ene-3,11,20-trione); 21-Hydroxypregnenolone (prebediolone; $3\beta$,21-dihydroxypregn-5-en-20-one); aldosterone ($11\beta$,21-dihydroxypregn-4-ene-3,18,20-trione); corticosterone (17-deoxycortisol; $11\beta$,21-dihydroxypregn-4-ene-3,20-dione); cortisol (hydrocortisone; $11\beta$,$17\alpha$,21-trihydroxypregn-4-ene-3,20-dione); cortisone ($17\alpha$,21-dihydroxypregn-4-ene-3,11,20-trione); pregnenolone (pregn-5-en-$3\beta$-ol-20-one); progesterone (pregn-4-ene-3,20-dione).

In some embodiments, a corticosteroid response may be a rise in a subject's blood glucocorticoid (glucocorticoidsteroid) level, e.g. a glucocorticoid response. A glucocorticoid is a corticosteroid that binds to the glucocorticoid receptor. A glucocorticoid response may comprise a rise in blood level of corticosterone or cortisol. In particular embodiments, a corticosteroid response may be a rise in cortisol, e.g. a cortisol response.

The phrase "a strong corticosteroid response" is used herein to refer to a corticosteroid response that is non-dose dependent, delayed, and has a high daily steroidal equivalent dose. Determining the characteristics of the desired corticosteroid response that is appropriate for the subject is a determination that a medical professional, with the ordinary level of skill expected of such a professional, can determine. Such a professional may consider the constitution of the subject, the disease, disorder, or condition that the subject has been diagnosed with or is at risk for, the previous treatments administered to the patient, and the current medications taken by the subject.

In some embodiments, a strong corticosteroid response is beneficial when the subject is responsive to steroid therapy. In contrast, in other embodiments, a strong corticosteroid response is not beneficial when the subject is substantially not responsive to steroid therapy. As used herein, "substantially not responsive" means that at typical dosages and administration schedules, a subject does not show improvement of one or more clinical factors.

### EXAMPLES

The following examples are included to demonstrate various embodiments of the present disclosure. It should be appreciated by those of skill in the art that the techniques disclosed in the examples that follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

## Example 1

Studies were conducted to discover the differences between repository corticotropin injection (RCI) (i.e. Acthar® gel) and synthetic adrenal corticotropin compositions (referred to as ACTH herein) on corticosterone levels in rats.

RCI (10, 40, or 400 IU/kg) or ACTH (0.6, 1.2, or 2.4 mg/kg) was administered to Sprague Dawely rats, plasma samples collected and analyzed for corticosterone.

RCI and ACTH peak corticosterone and area under the curve (AUC) were evaluated. RCI-induced corticosterone rapidly peaked between 2-8 hrs and was dependent on the dose. ACTH displays a delay in the peak time (8-24 hours) and was not dose dependent. When compared to the high dose ACTH, RCI reduced the AUC by $50.0 \pm 2.6\%$, $45.5 \pm 5.6\%$ and $27.3 \pm 12.5\%$ respectively for 10, 40, and 400 IU/kg. However, there was no significant reduction between any of the ACTH doses tested.

## Example 2

A phase 1 study in healthy adult subjects directly compared the effects of clinically relevant doses (used for nephrotic syndrome) of Acthar Gel and synthetic $ACTH_{1-24}$ depot on endogenous cortisol production. Oral doses of methylprednisolone used for nephrotic syndrome were utilized to estimate the prednisone equivalent doses of Acthar Gel and synthetic $ACTH_{1-24}$ depot. This study also characterized the PK and safety of each drug.

## Materials and Methods

### Study Objectives

The objectives of this study were as follows: to assess the PK of Acthar Gel, synthetic $ACTH_{1-24}$ depot, and methylprednisolone; to compare the PD of Acthar Gel with synthetic $ACTH_{1-24}$ depot; to estimate prednisone equivalent doses of Acthar Gel and synthetic $ACTH_{1-24}$ depot; and to assess the safety of Acthar Gel, synthetic $ACTH_{1-24}$ depot, and methylprednisolone.

### Subjects

The study was conducted at PRA Health Sciences, Inc. (Salt Lake City, Utah). The study protocol and informed consent forms were reviewed and approved by an institutional review board before any subjects provided consent. Subjects gave written informed consent prior to the initiation of study procedures. The study complied with local and federal regulations and with ethical guidelines outlined in the Declaration of Helsinki.

Healthy subjects were included if they were aged 18 to 50 years at the screening visit; weighed greater than 50 kg at the screening and check-in visits; had a body mass index $\geq 18.5$ and $\leq 30$ kg/m$^2$ at the screening and check-in visits; and were healthy as determined by medical history, physical examination, and clinical laboratory measurements. Subjects were excluded if they had a history of adverse or allergic reactions to any study drug or similar products; used prescription or over-the-counter oral, injected, inhaled, or topical corticosteroids within 30 days of the screening visit; recently donated or received blood products; had major surgery within 3 months or serious illness requiring hospitalization within 6 months of the check-in visit; had current or recent cardiovascular, endocrine, gastrointestinal, hematologic, hepatic, immunologic, infectious, musculoskeletal, neurologic, psychiatric, pulmonary, or renal disorders; or had current or recent alcohol, drug, or nicotine abuse.

### Study Design

This phase 1, single-center, open-label, randomized, parallel group study assigned 48 subjects to one of three study drugs (Figure 1): 16 subjects received Acthar Gel 80 IU subcutaneously (SC) twice weekly (BIW) on study Day 1 and Day 4; 16 subjects received synthetic $ACTH_{1-24}$ depot 1 mg SC BIW on study Day 1 and Day 4; and 16 subjects received oral methylprednisolone 32 mg (two 16-mg tablets) once daily on study Day 1 through Day 6. Baseline demographics were collected during the screening period between study Day -28 and Day -3. Subjects remained at the clinic from 2 days before the first dose of study drug (Day -2) until study Day 8.

Oral methylprednisolone is a corticosteroid commonly used for the treatment of inflammatory disorders such as nephrotic syndrome [30]. The drug was included in this study in order to determine its cortisol-equivalent exposure (steroidogenic equivalence) and for estimation of the steroidal dose equivalence of Acthar Gel and synthetic $ACTH_{1-24}$ depot when used at therapeutically equivalent doses. Therefore, the dosing regimens for Acthar Gel and methylprednisolone in this study were chosen to match the typical maintenance doses for each drug when used to treat nephrotic syndrome [8, 12, 30]. The dosing regimen for synthetic $ACTH_{1-24}$ depot was chosen to match the maintenance dose of the Synacthen® Depot product used outside of the US for nephrotic syndrome [27, 30].

**Analytical Methods**

Serum samples for total cortisol and plasma samples for free cortisol, total methylprednisolone, free methylprednisolone, and $ACTH_{1-24}$ were analyzed by Syneos Health (Quebec City, Quebec, Canada); plasma samples for N25D porcine $ACTH_{1-39}$ were analyzed by QPS Netherlands BV (Groningen, Netherlands). Concentrations for all analytes were determined using liquid chromatography-tandem mass spectrometry methods that were validated according to the US FDA Bioanalytical Method Validation Guidance (May 2018).

*N25D porcine $ACTH_{1-39}$*

The plasma N-25 deamidated (N25D) porcine adrenocorticotropic hormone $(ACTH)_{1-39}$ concentrations were determined using a validated liquid chromatography-tandem mass spectrometry (LC-MS/MS) method developed by QPS Netherlands BV (Groningen, Netherlands). The assay consisted of isolation by immune extraction using customized MSIA™ D.A.R.T.s.™ (Thermo Scientific™) and subsequent analysis of the extract with LC-MS/MS (Triple Quad™ 6500 LC-MS/MS detector, Sciex™) using positive electrospray ionization and multiple reaction monitoring (precursor/product ion, m/z: 762.2/881.6 for N25D porcine $ACTH_{1-39}$ and 764.5/884.3 for rACTH, which was included as internal standard). Quantitative analysis was accomplished with an Xbridge BEH300 C18 3.5 $\mu$m 2.1×50 mm column (Waters Corporation). The mobile phases were 0.5% formic acid and 0.001% trifluoroacetic acid (TFA) in Milli-Q (A) and acetonitrile (B). The LC was run on gradient mode. The calibration range for N25D porcine $ACTH_{1-39}$ was 10.0 to 240 pg/mL with lower limit of quantitation (LLOQ) of 10.0 pg/mL. The inter- and intra-run precision established during method validation (relative standard deviation [SD]) were ≤15.4% and ≤13.1%, respectively, while the intra-run accuracy was 84.9% to 97.4%.

*$ACTH_{1-24}$*

The plasma $ACTH_{1-24}$ concentrations were determined using a validated LC-MS/MS method developed by Syneos Health (Quebec City, Quebec, Canada). The assay consisted of positive electrospray ionization and multiple reaction monitoring. A sum of two transitions for $ACTH_{1-24}$ was used (precursor/product ion m/z: 490/537 and 588/670 for $ACTH_{1-24}$; 591/676 for $ACTH_{1-24}$ $d_{16}$, which was included as internal standard). Extraction of $ACTH_{1-24}$ from the plasma samples was done using an immunocapture technique with mouse ACTH monoclonal antibody immobilized on magnetic beads. The purified extract was injected into an ultra-performance liquid chromatography system with an Onyx Monolithic C18 (50 × 2.0 mm) column and a Xevo TQS tandem mass spectrometer as detector. The mobile phases were Milli-Q type water/acetic acid with TFA (A) and methanol/acetonitrile (B). The LC was run on gradient mode. The calibration range for plasma $ACTH_{1-24}$ concentration was 10.0 to 2000 pg/mL with LLOQ of 10.0 pg/mL. The inter- and intra-run-precision (relative SD) were ≤14% and 5%, respectively, while the intra-run accuracy was 88% to 104%.

*Plasma Total and Free Methylprednisolone*

The plasma total and free methylprednisolone were determined using a validated LC-MS/MS method developed by Syneos Health (Quebec City, Quebec, Canada). The LC-MS/MS methods for plasma total and free methylprednisolone were conducted using an Atmospheric Pressure Ionization (API) 5000 mass spectrometer (AB Sciex, Toronto, Canada) with positive electrospray ionization and multiple reaction monitoring (precursor/product ion m/z: 375/161 for methylprednisolone; 381/363 for methylprednisolone-$d_6$, which was included as internal standard). Quantitative analysis was accomplished on an ACE Excel 2 C18-PFP (50 x 3.0 mm, 2 $\mu$m) column. The mobile phases were Milli-Q type water/methanol with ammonium formate and formic acid (in different proportions). The LC was run on isocratic mode followed by a column flush. The calibration range for plasma total methylprednisolone concentration was 0.500 to 500 ng/mL with LLOQ of 0.500 ng/mL. The inter- and intra-run-precision (relative SD) were ≤7% and 8%, while the intra-run accuracy was 100% to 112%. The calibration range for plasma free cortisol concentration was 200 to 100,000 pg/mL with LLOQ of 2000 pg/mL. The inter- and intra-run-precision (relative SD) were ≤18.5% and 3%, respectively, while the intra-run accuracy was 94% to 101%.

*Serum Total and Plasma Free Cortisol*

The total and free cortisol serum concentrations were determined using validated LC-MS/MS methods developed by Syneos Health (Quebec City, Quebec, Canada). The LC-MS/MS methods were conducted using API 4000 mass spectrometer (AB Sciex, Toronto, Canada) for serum total cortisol concentrations and using an API 5000 mass spectrometer (AB Sciex) for plasma free cortisol concentrations. The LC-MS/MS methods for total and free cortisol used positive electrospray ionization and multiple reaction monitoring (precursor/product ion m/z: 363/121 for cortisol; 367/121 for cortisol-$d_4$, which was included as internal standard). Quantitative analyses for both serum total and plasma free cortisol were accomplished on an Atlantis dC18 (50 x 4.6 mm, 3 $\mu$m) column. The mobile phases were Milli-Q type water/methanol with ammonium formate (A) and methanol (B). The LC was run on isocratic mode followed by column flush. The calibration range for serum total cortisol concentrations was 0.500 to 500 ng/mL with LLOQ of 0.500 ng/mL. The inter- and intra-run-precision (relative SD) were ≤3% and 2%, while the intra-run accuracy was 96% to 101%. The calibration range for plasma free cortisol concentration was 100 pg/mL to 100,000 pg/mL with LLOQ of 100 pg/mL. The inter- and intra-run-precision (relative SD) were ≤12% and 2%, respectively, while the intra-run accuracy was 99% to 102%.

**Pharmacokinetic Analyses**

Serial whole blood samples were drawn from each subject over 24 hours after Acthar Gel, synthetic $ACTH_{1-24}$ depot, and methylprednisolone administration on study Day 1 and Day 4 for determination of plasma concentrations of N25D porcine $ACTH_{1-39}$ (a major component of the complex mixture of Acthar Gel), $ACTH_{1-24}$, and total and free methylprednisolone, respectively. All subjects whose PK profile contained at least 4 consecutive quantifiable concentrations were included in the PK population.

A major component in the complex mixture of Acthar Gel is N25D porcine $ACTH_{1-39}$; this component was utilized as the plasma marker for estimation of the pharmacokinetic (PK) parameters for Acthar Gel. All subjects whose PK profile contained at least 4 consecutive quantifiable plasma concentrations of N25D porcine $ACTH_{1-39}$, $ACTH_{1-24}$, or total and free methylprednisolone were in the PK population.

The following PK parameters for N25D porcine $ACTH_{1-39}$, $ACTH_{1-24}$, and total and free methylprednisolone were estimated: maximum plasma drug concentration ($C_{max}$) and the corresponding time ($T_{max}$); area under the plasma drug concentration-time curve from time zero to 24 hours, to the last quantifiable time, and to infinity ($AUC_{0-24}$, $AUC_{0-last}$, and $AUC_{0-inf}$, respectively); accumulation ratio ($RAUC_{0-24}$), calculated by dividing $AUC_{0-24}$ on Day 4 by $AUC_{0-24}$ on Day 1; and terminal elimination half-life (t½), calculated by dividing natural log (ln)2 (or 0.693) by the elimination rate constant ($K_{el}$).

PK parameters were estimated using actual blood collection times by noncompartmental methods using Phoenix® WinNonlin® 8.1. (Certara USA, Inc.). $AUC_{0-24}$, $AUC_{0-last}$, and $AUC_{0-inf}$ were calculated using the linear log trapezoidal rule. $K_{el}$ was calculated using linear regression on the terminal slope of the ln-concentration versus time curve. At least 3 time points (excluding $C_{max}$) and $r^2$ ≥0.90 were required to calculate and retain $K_{el}$ and its associated parameters (t½ and $AUC_{0-inf}$). In addition, an extrapolated %AUC of <20% was needed to retain t½ and $AUC_{0-inf}$.

**Pharmacodynamic Analyses**

*Plasma Free and Serum Total Cortisol*

Whole blood samples were drawn from each subject at predefined time points after Acthar Gel, synthetic $ACTH_{1-24}$ depot, and methylprednisolone administration and were time-matched on study Day -1, Day 1, and Day 4 for determination of plasma free and serum total cortisol concentrations. All subjects whose PD profile contained at least 4 consecutive quantifiable concentrations of total and free cortisol on both Day -1 and Day 1 were included in the PD population.

The following PD parameters for plasma free and serum total cortisol were estimated for Acthar Gel and synthetic $ACTH_{1-24}$ depot: maximum effect at 24 hours and 48 hours ($E_{max24}$ and $E_{max48}$, respectively); time to $E_{max24}$ and $E_{max48}$ ($TE_{max24}$ and $TE_{max48}$, respectively); area under the effect curve for the concentration-time profile from time zero to 24 hours and to 48 hours ($AUEC_{24}$ and $AUEC_{48}$, respectively); and baseline-corrected values for each ($E_{max24}[BA]$, $E_{max48}[BA]$, $TE_{max24}[BA]$, $TE_{max48}[BA]$, $AUEC_{24}[BA]$, and $AUEC_{48}[BA]$, respectively). $AUEC_{24}[BA]$ and $AUEC_{48}[BA]$ for Day 1 and Day 4 were calculated by subtracting the $AUEC_{24}$ and $AUEC_{48}$ on Day -1 from the respective Day 1 and Day 4 estimates for each study drug. $AUEC_{24}[BA]$ and $AUEC_{48}[BA]$ for serum total and plasma free cortisol after administration of synthetic $ACTH_{1-24}$ depot were compared to $AUEC_{24}[BA]$ and $AUEC_{48}[BA]$ for total and free cortisol after administration of Acthar Gel, respectively.

PD parameters were estimated using actual blood collection times by noncompartmental methods using Phoenix® WinNonlin® 8.1 (Certara USA, Inc.). AUEC$_{24}$ and AUEC$_{48}$ were calculated using the linear trapezoidal with linear interpolation rule.

*Cortisol-Equivalent Exposure (Steroidogenic Exposure)*

Whole blood samples were drawn from each subject at predefined time points over 24 hours after oral methylprednisolone administration on study Day 1 and Day 4 for determination of plasma total and free methylprednisolone concentrations. All subjects who were in both the PK and PD populations were included in the cortisol-equivalent exposure population.

Because oral methylprednisolone is known to suppress endogenous cortisol production [31], PD parameters for serum total and plasma free cortisol after administration of methylprednisolone were not calculated. Instead, the cortisol-equivalent (steroidogenic) concentration (CSG) of methylprednisolone was estimated. As a corticosteroid, methylprednisolone exhibits systemic action similar to cortisol; therefore, the steroidogenic concentration of methylprednisolone took into account the effects arising from endogenous cortisol (Conc$_{Cortisol}$) and from methylprednisolone itself. Additionally, methylprednisolone is considered 5 times more potent than cortisol (ie, every 1 mg of methylprednisolone is equivalent to 5 mg of cortisol) [4]. Therefore, a conversion factor of 5 was applied to the methylprednisolone concentration (Conc$_{MPD}$) [32]. The following equation was used to estimate the steroidogenic concentration of methylprednisolone (CSG):

$$CSG = (5 \times Conc_{MPD}) + Conc_{Cortisol}$$

CSG was used to estimate the following PD parameters for the steroidogenic concentration of plasma total and free methylprednisolone: maximum observed effect for the steroidogenic concentration (CSG) concentration-time profile (E$_{maxSG}$), time of E$_{maxSG}$ (T$_{maxSG}$), and area under the effect curve for the CSG concentration-time profile from time 0 to 24 hours (AUEC$_{24SG}$). Baseline-corrected AUEC$_{24SG}$ (AUEC$_{24SG}$[BA]) was calculated by subtracting the baseline (Day -1) cortisol concentrations from the time-matched Day 1 steroidogenic concentration.

*Estimated Prednisone Equivalent Doses*

To estimate the prednisone equivalence of the studied doses of Acthar Gel and synthetic ACTH$_{1-24}$ depot, we multiplied the plasma free cortisol AUEC$_{24}$[BA] on Day 4 for Acthar Gel and synthetic ACTH$_{1-24}$ depot by 2 (the number of weekly doses of each drug).

This value was divided by the free cortisol AUEC$_{24SG}$[BA] on Day 4 for 32 mg of methylprednisolone, then multiplied by the dose of methylprednisolone (32 mg). The prednisone equivalent dose was then calculated by multiplying this value by 1.25 (ie, every 1 mg of methylprednisolone is equivalent to 1.25 mg of prednisone) as provided in the equation below:

$$\text{Prednisone equivalent dose} =$$
$$[(\text{Day 4 free cortisol AUEC}_{24}[BA] \times 2) / (\text{Day 4 free cortisol AUEC}_{24SG}[BA])]$$
$$\times 32 \text{ mg} \times 1.25$$

**Safety Analyses**

All subjects who received at least one dose of the study drug were in the safety population. Safety was assessed via adverse events (AEs), treatment-emergent AEs (TEAEs; events that occurred after the first dose of study drug and through study completion), serious TEAEs (TESAEs), physical examinations, vital signs, 12-lead electrocardiograms (ECGs), and clinical laboratory test findings collected throughout the study.

**Statistical Analyses**

All PK parameters, PD parameters, and safety results were summarized descriptively. For plasma free and serum total cortisol exposure, the changes from baseline in AUEC$_{24}$ and AUEC$_{48}$ after administration of Acthar Gel and synthetic ACTH$_{1-24}$ depot were compared using an analysis of variance (ANOVA). The ANOVA model, with study drug as a fixed effect and subject as a random effect, was performed on Day 1 and Day 4 for AUEC$_{24}$[BA] and AUEC$_{48}$[BA] after natural log (ln) transformation. The results were back-transformed to the original scale. The ratio (percentage) of geometric least squares (LS) means, corresponding 90% confidence intervals, and *p* values are presented.

Results

**Subjects**

Baseline subject demographics are presented in Table 1. Subjects were predominantly male (75.0%) and White (83.3%) and were not of Hispanic or Latino ethnicity (83.3%). The mean age, body weight, and body mass index were similar among the 3 study drug groups.

**Table 1.** Baseline[a] Subject Demographics

| | Acthar Gel 80 IU SC BIW | Synthetic ACTH$_{1-24}$ depot 1 mg SC BIW | Methylprednisolone tablets 32 mg orally daily |
|---|---|---|---|
| | **n=16** | **n=16** | **n=16** |
| **Age, mean (SD), y** | 29.1 (5.5) | 25.9 (4.7) | 27.9 (6.2) |
| **Sex, No. (%)** | | | |
| Female | 3 (18.8) | 3 (18.3) | 6 (37.5) |
| Male | 13 (81.3) | 13 (81.3) | 10 (62.5) |
| **Race, No. (%)** | | | |
| American Indian or Alaska Native | 2 (12.5) | 0 | 1(6.3) |
| Asian | 1(6.3) | 0 | 0 |
| Black or African American | 1(6.3) | 1(6.3) | 1 (6.3) |
| White | 12 (75.0) | 14 (87.5) | 14 (87.5) |
| Other | 0 | 1 (6.3) | 0 |
| **Ethnicity, No. (%)** | | | |
| Hispanic or Latino | 3 (18.8) | 4 (25.0) | 1 (6.3) |
| Not Hispanic or Latino | 13 (81.3) | 12 (75.0) | 15 (93.8) |
| Weight, mean (SD), kg | 74.9 (9.7) | 77.6 (12.6) | 78.7 (15.2) |
| **BMI, mean (SD), kg/m$^2$** | 24.1 (2.4) | 24.4 (3.1) | 25.1 (2.9) |
| Collected during the screening period between study Day -28 and Day -3. Abbreviations: ACTH$_{1-24}$, the first 24 amino acids of adrenocorticotropic hormone; BIW, twice weekly; BMI, body mass index; SC, subcutaneous; SD, standard deviation. | | | |

**Pharmacokinetics**

*N25D porcine ACTH$_{1-39}$*

Plasma N25D porcine ACTH$_{1-39}$, a major component in the complex mixture of Acthar Gel, was utilized as the plasma marker. Mean plasma N25D porcine ACTH$_{1-39}$ concentrations increased rapidly following subcutaneous (SC) injection, with the mean peak N25D porcine ACTH$_{1-39}$ concentration occurring at 1 hour after the first dose (Figure 5). On Day 4, the mean peak plasma N25D porcine ACTH$_{1-39}$ concentration occurred at 1 hour and was slightly lower than that observed on Day 1.

The estimated PK parameters for plasma N25D porcine ACTH$_{1-39}$ are provided in Table 2. After SC administration of Acthar Gel to healthy subjects, N25D porcine ACTH$_{1-39}$ was absorbed rapidly from the injection site with a median $T_{max}$ of 2.0 hours and 1.1 hours after single and multiple dosing, respectively. The $t_{1/2}$ could not be calculated for 6 subjects on Day 1 and for 9 subjects on Day 4, as 3 consecutive quantifiable concentrations were not observed after the peak concentration. The mean $t_{1/2}$ for N25D porcine $ACTH_{1-39}$ following single- and multiple-dose administration was 2.6 hours and 3.4 hours, respectively. As quantifiable plasma N25D porcine $ACTH_{1-39}$ concentrations were not observed after 12 hours on Days 1 and 4, $AUC_{0-24}$ and $RAUC_{0-24}$ were not estimated; $AUC_{0-last}$ was reported instead of $AUC_{0-24}$ for Day 4.

**Table 2.** Mean (SD)[a] N25D porcine $ACTH_{1-39}$ Plasma Pharmacokinetic Parameters After Twice-Weekly Administration of Acthar Gel 80 IU SC, Study Day 1 and Day 4[b]

| PK parameter, unit | Day 1 | Day 4 |
|---|---|---|
| $C_{max}$, pg/mL | n=16 293.0 (401.0) | n=16 203.0 (327.0) |
| $T_{max}$, h | n=16 2.0 (0.3, 8.0) | n=16 1.1 (0.3, 4.0) |
| $t_{1/2}$, h | n=10 2.6(0.6) | n=7 3.4 (0.6) |
| $AUC_{0-last}$, h*pg/mL | n=16 797.0 (396.0) | n=16 722.0 (440.0) |
| $AUC_{0-inf}$, h*pg/mL | n=10 1020.0 (406.0) | NA |

[a]$T_{max}$ is presented as median (minimum, maximum). [b]PK parameters were estimated for subjects who had at least 4 consecutive quantifiable plasma concentrations for N25D porcine $ACTH_{1-39}$. Abbreviations: $ACTH_{1-39}$, the first 39 amino acids of adrenocorticotropic hormone; $AUC_{0-inf}$, area under the concentration-time curve from time 0 to infinity; $AUC_{0-last}$, area under the concentration-time curve from time 0 to the time of the last quantifiable concentration; $C_{max}$, observed peak plasma concentration; N25D, N-25 deamidated; NA, not applicable; PK, pharmacokinetic; SC, subcutaneously; SD, standard deviation; $t_{1/2}$, terminal elimination half-life; $T_{max}$, time of maximum observed plasma concentration.

$ACTH_{1-24}$

Mean plasma $ACTH_{1-24}$ concentrations increased rapidly following SC injection, with peak concentration occurring at 15 minutes after dosing (Figure 6). The mean peak plasma $ACTH_{1-24}$ concentration on Day 4 occurred at 15 minutes and was slightly lower than the mean peak $ACTH_{1-24}$ concentration observed on Day 1.

The plasma PK parameters that were estimated for $ACTH_{1-24}$ are provided in Table 3. After SC administration of synthetic $ACTH_{1-24}$ depot, $ACTH_{1-24}$ was absorbed rapidly from the injection site, with a median $T_{max}$ of 0.3 hours after single and multiple dosing. Fourteen subjects had quantifiable concentrations for up to 8 hours, and 2 subjects had quantifiable concentrations for up to 12 hours. Except for 1 and 2 subjects on Day 1 and Day 4, respectively, $t_{1/2}$ could not be calculated, as 3 consecutive quantifiable plasma $ACTH_{1-24}$ concentrations were not observed after the peak concentration. The $t_{1/2}$ of $ACTH_{1-24}$ following a single dose was 2.8 hours, and the mean $t_{1/2}$ following multiple doses was 2.8 hours. Because quantifiable plasma $ACTH_{1-24}$ concentrations were not observed after 12 hours on Days 1 and 4, $AUC_{0-24}$ and $RAUC_{0-24}$ were not estimated; $AUC_{0-last}$ was reported instead of $AUC_{0-24}$ for Day 4.

**Table 3.** Mean (SD)[a] $ACTH_{1-24}$ Plasma Pharmacokinetic Parameters After Twice-Weekly Administration of Synthetic $ACTH_{1-24}$ Depot 1 mg SC, Study Day 1 and Day 4[b]

| PK parameter, unit | Day 1 | Day 4 |
|---|---|---|
| $C_{max}$, pg/mL | n=13 103.0 (103.0) | n=13 86.1 (75.0) |
| $T_{max}$, h | n=13 0.3 (0.3, 6.0) | n=13 0.3 (0.3, 4.0) |
| $t_{1/2}$, h | n=1 2.8 (NA) | n=2 2.4 (0.5) |
| $AUC_{0-last}$, h*pg/mL | n=13 259.0 (231.0) | n=13 227.0 (184.0) |
| $AUC_{0-inf}$, h*pg/mL | n=1 366.0 (NA) | NA |

[a]$T_{max}$ is presented as median (minimum, maximum); mean is presented when n≥2. [b]PK parameters were estimated for subjects who had at least 4 consecutive quantifiable plasma concentrations for $ACTH_{1-24}$.

Abbreviations: $ACTH_{1-24}$, the first 24 amino acids of adrenocorticotropic hormone; $AUC_{0-inf}$, area under the concentration-time curve from time 0 to infinity; $AUC_{0-last}$, area under the concentration-time curve from time 0 to the time of the last quantifiable concentration; $C_{max}$, observed peak plasma concentration; NA, not applicable; PK, pharmacokinetic; SC, subcutaneously; SD, standard deviation; $t_{1/2}$, terminal elimination half-life; $T_{max}$, time of maximum observed plasma concentration.

*Plasma Total and Free Methylprednisolone*

The mean plasma total methylprednisolone concentration peaked around 2 hours after oral dosing and declined rapidly (data not shown). On Day 4, the mean plasma total methylprednisolone concentration was slightly lower than on Day 1. Similar to the plasma total methylprednisolone concentration, the mean plasma free methylprednisolone concentration peaked at 2 hours after oral dosing and declined rapidly (data not shown). On Day 4, the mean plasma total methylprednisolone concentration was slightly lower than on Day 1.

The estimated PK parameters for plasma total and free methylprednisolone are provided in Table 4. After oral administration of methylprednisolone tablets, total methylprednisolone was absorbed with a median $T_{max}$ of 2 hours after single and multiple doses. The mean $t_{1/2}$ of methylprednisolone following single and multiple doses was quite similar (2.2 hours and 2.4 hours, respectively). No accumulation of methylprednisolone was seen with mean $RAUC_{0-24}$ of 0.9. Similar to plasma total methylprednisolone, the median $T_{max}$ for plasma free methylprednisolone was 2 hours for both Day 1 and Day 4. The mean $t_{1/2}$ for plasma free methylprednisolone (1.9 hours and 2.0 hours for Day 1 and Day 4, respectively) was quite similar to total methylprednisolone (2.2 hours and 2.4 hours for Day 1 and Day 4, respectively). The mean exposure of plasma free methylprednisolone was about 8% of the mean exposure of plasma total methylprednisolone on both days. Only one subject had a quantifiable plasma free methylprednisolone concentration until 24 hours postdose. $RAUC_{0-24}$ for this subject was 0.8.

Plasma-concentration time profiles for free and total methylprednisolone and PK parameters estimated from these plasma concentrations were consistent with those reported in the literature.

Table 4. Mean (SD)[a] Plasma Total and Free Methylprednisolone Pharmacokinetic Parameters After Administration of Oral Methylprednisolone 32 mg Daily, Study Day 1 and Day 4[b]

| PK parameter, unit | Day 1 | Day 4 |
|---|---|---|
| **Total methylprednisolone** | | |
| $C_{max}$, ng/mL | n=16 198.0 (51.4) | n=14 188.0 (40.0) |
| $T_{max}$, h | n=16 2.0 (2.0, 4.0) | n=14 2.0 (1.0, 4.0) |
| $t_{1/2}$, h | n=16 2.2 (0.6) | n=14 2.4 (0.7) |
| $AUC_{0-24}$, h*ng/mL | n=8 1330.0 (267.0) | n=8 1110.0 (221.0) |
| $AUC_{0-last}$ h*ng/mL | n=16 1020.0 (381.0) | n=14 919.0 (297.0) |
| $AUC_{0-inf}$, h*ng/mL | n=16 1030.0 (380.0) | NA |
| $RAUC_{0-24}$ | NA | n=7 0.9 (0.1) |
| **Free methylprednisolone[b]** | | |
| $C_{max}$, ng/mL | n=16 24.6 (7.4) | n=15 21.9 (5.4) |
| $T_{max}$, h | n=16 2.0 (1.0, 4.0) | n=15 2.0 (1.0, 4.0) |
| $t_{1/2}$, h | n=16 1.9 (0.5) | n=13 2.0 (0.5) |
| $AUC_{0-24}$, h*ng/mL | n=1 176.0 (NA) | n=1 145.0 (NA) |
| $AUC_{0-last}$ h*ng/mL | n=16 104.0 (35.2) | n=15 89.5 (27.3) |
| $AUC_{0-inf}$, h*ng/mL | n=16 106.0 (36.0) | NA |
| $RAUC_{0-24}$ | NA | n=1 0.8 (NA) |

[a]$T_{max}$ is presented as median (minimum, maximum); mean is presented when n≥2. [b]PK parameters were estimated for subjects who had at least 4 consecutive quantifiable plasma concentrations for total and free methylprednisolone. Abbreviations: $AUC_{0-24}$, area under the concentration-time curve from time 0 to 24 hours postdose; $AUC_{0-inf}$, area under the concentration-time curve from time 0 to infinity; $AUC_{0-last}$ area under the concentration-time curve from time 0 to the time of the last quantifiable concentration; $C_{max}$, observed peak plasma concentration; NA, not applicable; PK, pharmacokinetic; $RAUC_{0-24}$, accumulation ratio; SD, standard deviation; $t_{1/2}$, terminal elimination half-life; $T_{max}$, time of maximum observed plasma concentration.

**Pharmacodynamics**

*Plasma Free and Serum Total Cortisol*

Because plasma free cortisol is pharmacologically active [33], we assessed free cortisol concentrations as an indicator of the physiologic effects of each study drug. The mean peak baseline-corrected free cortisol concentrations on study Day 1 (Figure 2A) and Day 4 (Figure 2B) were about 2-fold lower after administration of clinically relevant doses of Acthar Gel compared to synthetic $ACTH_{1-24}$ depot. Following a single dose (Day 1) and multiple doses (Day 4) of Acthar Gel, the free cortisol concentrations returned to baseline by 24 hours postdose, whereas free cortisol concentrations were observed for up to 48 hours after administration of synthetic $ACTH_{1-24}$ depot. The mean peak baseline-corrected serum total cortisol concentrations on study Day 1 (Figure 3A) and Day 4 (Figure 3B) were about 2-fold lower after administration of clinically relevant doses of Acthar Gel compared to synthetic $ACTH_{1-24}$ depot. At 24 hours after administration of Acthar Gel on both Day 1 and Day 4, the baseline-corrected total and free cortisol levels were negative because the observed total and free cortisol levels at baseline were higher than the total and free cortisol levels observed at this time point. Plasma total and free cortisol concentrations after administration of oral methylprednisolone were consistent with its known adrenal suppression (data not shown) [31].

The mean plasma free cortisol $AUEC_{24}[BA]$ and $AUEC_{48}[BA]$ were about 3-fold lower for Acthar Gel compared to synthetic $ACTH_{1-24}$ depot on Day 1 (Table 2). On Day 4, mean free cortisol $AUEC_{24}[BA]$ and $AUEC_{48}[BA]$ were about 4-fold and 5-fold lower, respectively, for Acthar Gel compared to synthetic $ACTH_{1-24}$ depot. Compared to Day 1, $E_{max24}[BA]$ was slightly higher on Day 4 for Acthar Gel. Compared to synthetic $ACTH_{1-24}$ depot, $E_{max24}[BA]$ was about 2-fold lower for Acthar Gel on Day 1 and Day 4. Compared to synthetic $ACTH_{1-24}$ depot, median $TE_{max24}[BA]$ occurred earlier for Acthar Gel on Day 1 but was similar on Day 4. Median $TE_{max24}[BA]$ and $TE_{max48}[BA]$ and mean $E_{max24}[BA]$ and $E_{max48}[BA]$ were the same as on Day 1 and Day 4 for Acthar Gel and synthetic $ACTH_{1-24}$ depot.

The mean serum total cortisol $AUEC_{24}[BA]$ and $AUEC_{48}[BA]$ values after a single dose (Day 1) of Acthar Gel were about 2-fold and 3-fold lower, respectively, for total cortisol $AUEC_{24}[BA]$ and $AUEC_{48}[BA]$ than for values observed after administration of synthetic $ACTH_{1-24}$ depot (Table 5). After multiple doses (Day 4), mean total cortisol $AUEC_{24}[BA]$ and $AUEC_{48}[BA]$ were about 3-fold lower for Acthar Gel than for synthetic $ACTH_{1-24}$ depot. Compared to Day 1, $E_{max24}[BA]$ was slightly higher on Day 4 for both Acthar Gel and synthetic $ACTH_{1-24}$ depot. Median $TE_{max24}[BA]$ was similar for Day 1 and Day 4 for both Acthar Gel and synthetic $ACTH_{1-24}$ depot but occurred earlier for Acthar Gel. Median $TE_{max24}[BA]$ and $TE_{max48}[BA]$ and mean $E_{max24}[BA]$ and $E_{max48}[BA]$ were the same as on Day 1 and Day 4 for Acthar Gel and synthetic $ACTH_{1-24}$ depot.

A comparison of total and free cortisol $AUEC_{24}[BA]$ and $AUEC_{48}[BA]$ using the LS geometric mean ratio (percentage) of synthetic $ACTH_{1-24}$ depot to Acthar Gel is shown in Figure 4. Statistically significant differences (p<0.0001) between synthetic $ACTH_{1-24}$ depot and Acthar Gel were observed on study Day 1 and Day 4 for both total and free $AUEC_{24}[BA]$ (Figure 4A) and $AUEC_{48}[BA]$ (Figure 4B), respectively.

**Table** 5. Mean (SD)[a] Plasma Free and Serum Total Cortisol Pharmacodynamic Parameters After Administration of Acthar Gel and Synthetic $ACTH_{1-24}$ Depot

| | Acthar Gel 80 IU SC BIW (N=16) | | Synthetic $ACTH_{1-24}$ depot 1 mg SC BIW (N=16) | |
|---|---|---|---|---|
| | Day 1 | Day 4 | Day 1 | Day 4 |
| Free cortisol[b,c] | n=14 | n=14 | n=13 | n=13 |
| Emax24, ng/mL | 36.0 (10.7) | 40.8 (17.6) | 61.8 (17.1) | 89.8 (24.8) |
| $E_{max24}[BA]$, ng/mL | 35.8 (10.7) | 40.6 (17.7) | 61.5 (17.0) | 89.5 (24.9) |
| TEmax24, h | 8.0 (2.0, 12.0) | 8.0 (2.0, 8.0) | 12.0 (8.0, 23.9) | 8.0 (8.0, 23.8) |
| $TE_{max24}[BA]$, h | 8.0 (2.0, 12.0) | 8.0 (2.0, 8.0) | 12.0 (8.0, 23.6) | 8.0 (8.0, 23.8) |
| $AUEC_{24}$, h*ng/mL | 466.0 (214.0) | 501.0 (261.0) | 1150.0 (312.0) | 1740.0 (522.0) |
| $AUEC_{24}[BA]$, h*ng/mL | 361.0 (164.0) | 395.0 (187.0) | 1140.0 (315.0) | 1730.0 (525.0) |
| $AUEC_{48}$, h*ng/mL | 566.0 (211.0) | 718.0 (414.0) | 1170.0 (656.0) | 2380.0 (797.0) |
| $AUEC_{48}[BA]$, h*ng/mL | 624.0 (359.0) | 490.0 (249.0) | 1680.0 (665.0) | 2340.0 (795.0) |

(continued)

| Total cortisol[c] | n=16 | n=16 | n=13 | n=13 |
|---|---|---|---|---|
| $E_{max24}$, ng/mL | 268.0 (42.4) | 284.0 (65.1) | 358.0 (63.4) | 416.0 (74.7) |
| $E_{max24}$[BA], ng/mL | 222.0 (47.0) | 240.0 (72.8) | 318.0 (61.0) | 380.0 (84.9) |
| $TE_{max24}$, h | 8.0 (1.0, 12.0) | 8.0 (2.0, 12.0) | 12.0 (8.0, 23.6) | 12.0 (8.0, 23.8) |
| $TE_{max24}$[BA], h | 8.0 (1.0, 12.1) | 8.0 (2.0, 12.0) | 12.0 (8.0, 23.6) | 12.0 (8.0, 23.8) |
| $AUEC_{24}$, h*ng/mL | 4440.0 (1110.0) | 4680.0 (1180.0) | 7520.0 (1160.0) | 8760.0 (1400.0) |
| $AUEC_{24}$[BA], h*ng/mL | 2480.0 (964.0) | 2410.0 (887.0) | 6010.0 (1310.0) | 7250.0 (1570.0) |
| $AUEC_{48}$, h*ng/mL | 5870.0 (1210.0) | 5790.0 (1260.0) | 12800.0 (2920.0) | 13700.0 (2560.0) |
| $AUEC_{48}$[BA], h*ng/mL | 3480.0 (1470.0) | 3550.0 (1730.0) | 9450.0 (3270.0) | 10400.0 (2720.0) |

[a]$TE_{max24}$ and $TE_{max24}$[BA] are presented as median (minimum, maximum). [b]In the Acthar Gel and synthetic $ACTH_{1-24}$ groups, 2 subjects and 3 subjects, respectively, did not have at least 4 consecutive quantifiable plasma concentrations of free cortisol on both Day -1 and Day 1 and therefore were not included in these analyses. [c]Results for $E_{max48}$ and $TE_{max48}$ are not reported, as they are the same as $E_{max24}$ and $TE_{max24}$, respectively.
Abbreviations: $ACTH_{1-24}$, the first 24 amino acids of adrenocorticotropic hormone; $AUEC_{24}$, area under the effect curve for the concentration-time profile from time 0 to 24 hours; $AUEC_{48}$, area under the effect curve for the concentration-time profile from time 0 to 48 hours; BA, baseline-corrected; BIW, twice weekly; $E_{max24}$, maximum observed effect at 24 hours; SC, subcutaneously; SD, standard deviation; $TE_{maX24}$, time to maximum observed effect at 24 hours.

*Cortisol-Equivalent Exposure (Steroidogenic Exposure)*

The mean (SD) plasma free cortisol-equivalent $AUEC_{24SG}$ of methylprednisolone on Day 1 and Day 4 was 572.0 h*ng/mL (184.0) and 492.0 h*ng/mL (136.0), respectively. The mean (SD) plasma total cortisol-equivalent $AUEC_{24SG}$ of methylprednisolone on Day 1 and Day 4 was 6480.0 h*ng/mL (1770.0) and 5440.0 h*ng/mL (1330.0), respectively. $E_{maxSG}$, $T_{maxSG}$, and $AUEC_{24SG}$[BA] for total and free methylprednisolone are provided in Table 7.

*Estimated Prednisone Equivalent Doses*

The estimated daily and weekly prednisone equivalent doses of Acthar Gel 80 IU SC BIW were about 4-fold lower than that of synthetic $ACTH_{1-24}$ depot 1 mg SC BIW (Table 6).

**Table** 6. Estimated Prednisone Equivalent Doses of Acthar Gel and Synthetic $ACTH_{1-24}$ Depot

| | Weekly prednisone dose (mg) | Daily prednisone dose (mg) |
|---|---|---|
| **Acthar Gel 80 IU SC BIW (160 IU per week)** | 67 | 10 |
| **Synthetic $ACTH_{1-24}$ depot 1 mg SC BIW (2 mg per week)** | 291 | 42 |
| Abbreviations: $ACTH_{1-24}$, the first 24 amino acids of adrenocorticotropic hormone; BIW, twice weekly; SC, subcutaneous. | | |

*Plasma Total and Free Methylprednisolone*

Pharmacodynamic parameters for plasma total and free methylprednisolone are provided in Table 7.

**Table** 7. Mean (SD)[a] Plasma Total and Free Methylprednisolone Pharmacodynamic Parameters After Daily Administration of Oral Methylprednisolone, Study Day 1 and Day 4[b]

| PD parameter, unit | Day 1 | Day 4 |
|---|---|---|
| **Total cortisol** | **n=16** | **n=15** |
| $T_{maxSG}$, h | 2.0 (2.0, 4.0) | 2.0 (1.0, 4.0) |
| EmaxSG, ng/mL | 1040.0 (251.0) | 959.0 (188.0) |
| $AUEC_{24SG}$[BA], h*ng/mL | 4860.0 (1680.0) | 4010.0 (1260.0) |
| **Free cortisol** | **n=16** | **n=15** |
| $T_{maxSG}$, h | 2.0 (1.0, 4.0) | 2.0 (1.0, 4.0) |
| EmaxSG, ng/mL | 124.0 (37.2) | 109.0 (26.7) |
| $AUEC_{24SG}$[BA], h*ng/mL | 553.0 (187.0) | 475.0 (140.0) |

[a]$T_{maxSG}$ is presented as median (minimum, maximum). [b]PD parameters were estimated for subjects who had at least 4 consecutive quantifiable plasma concentrations for total and free cortisol.

Abbreviations: $AUEC_{24SG}$, area under the effect curve for the steroidogenic concentration-time profile from time 0 to 24 hours; BA, baseline-corrected; $E_{maxSG}$, maximum observed effect for the steroidogenic concentration-time profile; PD, pharmacodynamic; SD, standard deviation; $T_{maxSG}$, time to maximum observed effect for the steroidogenic concentration-time profile.

**Safety**

All 48 subjects received at least one dose of Acthar Gel, synthetic $ACTH_{1-24}$ depot, or oral methylprednisolone. Most subjects (n=39; 81%) reported at least one AE. Fewer subjects in the Acthar Gel group reported AEs than did subjects in either of the other 2 study drug groups (Table 8). The most common TEAEs in the Acthar Gel group were headache and injection site reaction; however, a lower incidence of injection site reactions was observed in the Acthar Gel group than in the synthetic $ACTH_{1-24}$ depot group. One subject in the synthetic $ACTH_{1-24}$ depot group experienced a TESAE of pulmonary embolism; no subjects in the Acthar Gel group or methylprednisolone group experienced any TESAEs.

No clinically significant changes from baseline in physical examination findings, vital signs, ECG findings, or clinical laboratory tests results, including serum chemistry, hematology, or urinalysis, were observed for subjects in any study drug group (data not shown).

**Table 8.** Adverse Events

| | Acthar Gel 80 IU SC BIW | Synthetic $ACTH_{1-24}$ depot 1 mg SC BIW | Methylprednisolone tablets 32 mg orally daily | All subjects |
|---|---|---|---|---|
| | **n=16** | **n=16** | **n=16** | **N=48** |
| **AEs, No.** | 22 | 55 | 17 | 94 |
| **Subjects with any TEAE, No. (%)** | 12 (75.0) | 15 (93.8) | 10 (62.5) | 37 (77.1) |
| **Most common TEAEs (≥4% in any study drug group), No. (%)** | | | | |
| Contusion | 1 (6.3) | 2 (12.5) | 1 (6.3) | 4 (8.3) |
| Dysuria | 0 | 2 (12.5) | 0 | 2(4.2) |
| Headache | 3 (18.8) | 1 (6.3) | 2 (12.5) | 6 (12.5) |
| Heart palpitations | 1 (6.3) | 4 (25.0) | 2 (12.5) | 7 (14.6) |
| Injection site reaction | 3 (18.8) | 12 (75.0) | 0 | 15 (31.0) |

(continued)

| Most common TEAEs (≥4% in any study drug group), No. (%) | | | | |
|---|---|---|---|---|
| Insomnia | 0 | 2 (12.5) | 0 | 2(4.2) |
| Somnolence | 2 (12.5) | 0 | 0 | 2(4.2) |
| **Any TESAE, No. (%)** | 0 | 1 (6.3) | 0 | 1 (2.1) |
| Pulmonary embolism | 0 | 1 (6.3) | 0 | 1 (2.1) |

Abbreviations: $ACTH_{1-24}$, the first 24 amino acids of adrenocorticotropic hormone; AE, adverse event; BIW, twice weekly; SC, subcutaneous; TEAE, treatment-emergent adverse event; TESAE, treatment-emergent serious adverse event.

## DISCUSSION

Since therapeutic use of ACTH analog formulations became widespread in the 1950s and 1960s, studies have demonstrated variable cortisol responses after administration of different ACTH products [25, 26, 34, 35]. This phase 1, single-center, open-label, randomized, parallel group study is the first to report a direct comparison of the cortisol responses of Acthar Gel and synthetic $ACTH_{1-24}$ depot.

Administration of doses of Acthar Gel and synthetic $ACTH_{1-24}$ depot used to treat nephrotic syndrome resulted in substantially different levels of endogenous cortisol production. After BIW SC administration of each drug, Acthar Gel induced greater than 2-fold lower mean peak baseline-corrected plasma free cortisol concentrations than synthetic $ACTH_{1-24}$ depot.

Acthar Gel also induced a 5-fold lower plasma free cortisol exposure than synthetic $ACTH_{1-24}$ depot after 2 doses. Further, the ratio of total and free cortisol exposure for synthetic $ACTH_{1-24}$ depot to Acthar Gel showed significantly lower total and free cortisol exposure after administration of Acthar Gel compared to synthetic $ACTH_{1-24}$ depot. Comparisons between total and free cortisol exposure for Acthar Gel and synthetic $ACTH_{1-24}$ depot with total and free cortisol-equivalent exposure of methylprednisolone would not be appropriate due to differences in dosing regimens. In this study, oral methylprednisolone was given once daily on study Days 1 through 6, while Acthar Gel and synthetic $ACTH_{1-24}$ depot were given as SC doses on study Day 1 and Day 4. However, these dosage regimens were consistent with clinically relevant maintenance doses used for each study drug when treating nephrotic syndrome [8, 12, 30].

Results from this study estimated a low daily steroidal equivalent dose for Acthar Gel, with 80 IU SC BIW corresponding to 10 mg/d of prednisone. In contrast, synthetic $ACTH_{1-24}$ 1 mg SC BIW was found to be comparable to estimated doses of prednisone that were about 4-fold higher than for Acthar Gel. A previous model-based simulation study found the steroidal equivalent dose of Acthar Gel to be about 7-fold lower than that of synthetic $ACTH_{1-24}$ depot at the same clinically relevant doses [29]. However, this simulation used data from two separate PK/PD studies and did not directly measure plasma free cortisol exposure after administration of synthetic $ACTH_{1-24}$ depot 1 mg SC BIW. Thus, the current findings reflect estimates of the actual in vivo steroidal exposure of these clinically relevant dosing regimens. Despite having a low estimated daily steroidal equivalent dose, Acthar Gel has previously demonstrated clinical efficacy in patients with inflammatory conditions that have shown a poor clinical response to corticosteroids [8-18]. This further supports a therapeutic effect of Acthar Gel that is independent of cortisol production and suggests Acthar Gel may be an efficacious alternative for patients that have become resistant to the effects of corticosteroids or are unable to tolerate their side effects.

Interestingly, lower endogenous cortisol production after administration of Acthar Gel was observed despite having a slightly higher mean elimination half-life compared to synthetic $ACTH_{1-24}$ depot after multiple doses. Synthetic $ACTH_{1-24}$ depot achieved rapid peak concentrations similar to Acthar Gel and rapidly eliminated with no quantifiable plasma concentrations observed after 8 hours postdose for 88% of the subjects. Further, mean plasma N25D porcine $ACTH_{1-39}$ (the PK marker for Acthar Gel) concentrations were higher than mean plasma $ACTH_{1-24}$ concentrations (Figures 5 and 6, respectively), yet Acthar Gel induced 2-fold less cortisol than synthetic $ACTH_{1-24}$ depot after multiple doses (Figure 2B). The lower endogenous cortisol response of Acthar Gel despite its having higher plasma concentrations may be explained by its complex mixture of peptides and the heterodimeric nature of MCRs [37].

Safety results were consistent with the known safety profile for Acthar Gel [19]. Fewer injection site reactions were observed in the Acthar Gel group than in the synthetic $ACTH_{1-24}$ depot group (18.8% and 75.0%, respectively). No TESAEs were reported in the Acthar Gel group; however, one subject receiving synthetic $ACTH_{1-24}$ depot experienced a TESAE of pulmonary embolism.

This study has several limitations. As an open-label study, subjects and investigators were aware of study drug assignments. The study was conducted in a small sample of healthy subjects; thus, the clinical relevance of these findings should be investigated in a larger sample of patients who have inflammatory conditions.

## CONCLUSIONS

Acthar Gel is a complex mixture of modified porcine ACTH and other related peptide analogs. At clinically relevant maintenance doses used for the treatment of nephrotic syndrome, Acthar Gel induced less endogenous cortisol production and had lower cortisol exposure and lower steroidal equivalent doses than synthetic $ACTH_{1-24}$ depot after BIW administration. These results are supported by the clinical efficacy of Acthar Gel in the treatment of inflammatory disorders that have previously demonstrated an inadequate response to corticosteroids [8-18]. The different PD properties observed for Acthar Gel and synthetic $ACTH_{1-24}$ depot in this study suggest that these products in the ACTH class are not interchangeable.

## REFERENCES

1. Maniadakis N, Toth E, Schiff M, Wang X, Nassim M, Szegvari B, et al. A targeted literature review examining biologic therapy compliance and persistence in chronic inflammatory diseases to identify the associated unmet needs, driving factors, and consequences. Adv Ther. 2018 Sep;35(9):1333-55. https://doi.org/ 10.1007/s12325-018-0759-0.

2. Selmi C, Generali E, Massarotti M, Bianchi G, Scire CA. New treatments for inflammatory rheumatic disease. Immunol Res. 2014 Dec;60(2-3):277-88. https://doi.org/ 10.1007/s12026-014-8565-5.

3. Williams DM. Clinical pharmacology of corticosteroids. Respir Care. 2018 Jun;63(6):655-70. https://doi.org/ 10.4187/respcare.06314.

4. Zoorob RJ, Cender D. A different look at corticosteroids. Am Fam Physician. 1998 Aug;58(2):443-50.

5. Waljee AK, Rogers MA, Lin P, Singal AG, Stein JD, Marks RM, et al. Short term use of oral corticosteroids and related harms among adults in the United States: population based cohort study. BMJ. 2017 Apr 12;357:j1415. https://doi.org/ 10.1136/bmj.j1415.

6. Barnes PJ, Adcock IM. Glucocorticoid resistance in inflammatory diseases. Lancet. 2009 May 30;373(9678):1905-17. https://doi.org/ 10.1016/S0140-6736(09)60326-3.

7. Wilkinson L, Verhoog NJD, Louw A. Disease- and treatment-associated acquired glucocorticoid resistance. Endocr Connect. 2018 Dec;7(12):R328-R49. https://doi.org/ 10.1530/EC-18-0421.

8. Bomback AS, Tumlin JA, Baranski J, Bourdeau JE, Besarab A, Appel AS, et al. Treatment of nephrotic syndrome with adrenocorticotropic hormone (ACTH) gel. Drug Des Devel Ther. 2011 Mar 14;5:147-53. https://doi.org/ 10.2147/DDDT.S17521.

9. Berkovich R, Agius MA. Mechanisms of action of ACTH in the management of relapsing forms of multiple sclerosis. Ther Adv Neurol Disord. 2014 Mar;7(2):83-96. https://doi.org/10.1177/1756285613518599.

10. Fiechtner JJ, Montroy T. Treatment of moderately to severely active systemic lupus erythematosus with adrenocorticotropic hormone: a single-site, open-label trial. Lupus. 2014 Aug;23(9):905-12. https://doi.org/10.1177/0961203314532562.

11. Furie R, Mitrane M, Zhao E, Das M, Li D, Becker PM. Efficacy and tolerability of repository corticotropin injection in patients with persistently active SLE: results of a phase 4, randomised, controlled pilot study. Lupus Sci Med. 2016;3(1):e000180. https://doi.org/ 10.1136/lupus-2016-000180.

12. Madan A, Mijovic-Das S, Stankovic A, Teehan G, Milward AS, Khastgir A. Acthar gel in the treatment of nephrotic syndrome: a multicenter retrospective case series. BMC Nephrol. 2016 Mar 31;17:37. https://doi.org/ 10.1186/s12882-016-0241-7.

13. Furie RA, Mitrane M, Zhao E, Becker PM. Repository corticotropin injection in patients with persistently active SLE requiring corticosteroids: post hoc analysis of results from a two-part, 52-week pilot study. Lupus Sci Med. 2017;4(1):e000240. https://doi.org/ 10.1136/lupus-2017-000240.

14. Alhamad T, Manllo Dieck J, Younus U, Matar D, Alasfar S, Vujjini V, et al. ACTH gel in resistant focal segmental glomerulosclerosis after kidney transplantation. Transplantation. 2019 Jan;103(1):202-9. https://doi.org/ 10.1097/TP.0000000000002320.

15. Grafals M, Sharfuddin A. Adrenocorticotropic hormone in the treatment of focal segmental glomerulosclerosis following kidney transplantation. Transplant Proc. 2019 Jul - Aug;51(6):1831-7. https://doi.org/ 10.1016/j.transproceed.2019.04.052.

16. Fleischmann R, Furst DE, Connolly-Strong E, Liu J, Zhu J, Brasington R. Repository corticotropin injection for active rheumatoid arthritis despite aggressive treatment: a randomized controlled withdrawal trial. Rheumatol Ther. 2020 Jun;7(2):327-44. https://doi.org/ 10.1007/s40744-020-00199-3.

17. Zand L, Canetta P, Lafayette R, Aslam N, Jan N, Sethi S, et al. An open-label pilot study of adrenocorticotrophic hormone in the treatment of IgA nephropathy at high risk of progression. Kidney Int Rep. 2020 Jan;5(1):58-65. https://doi.org/ 10.1016/j.ekir.2019.10.007.

18. Askanase AD, Zhao E, Zhu J, Bilyk R, Furie RA. Repository corticotropin injection for persistently active systemic lupus erythematosus: results from a phase 4, multicenter, randomized, double-blind, placebo-controlled trial. Rheumatol Ther. 2020 Sep 29;7(4):839-908. https://doi.org/ 10.1007/s40744-020-00236-1.

19. Acthar Gel [package insert]. Mallinckrodt Pharmaceuticals; 2021.

20. Olsen NJ, Decker DA, Higgins P, Becker PM, McAloose CA, Benko AL, et al. Direct effects of HP Acthar Gel on human B lymphocyte activation in vitro. Arthritis Res Ther. 2015 Oct 27;17:300. https://doi.org/ 10.1186/s13075-015-0823-y.

21. Healy LM, Jang JH, Lin YH, Rao V, Antel JP, Wright D. Melanocortin receptor mediated anti-inflammatory effect of repository corticotropin injection on human monocyte-derived macrophages [ECTRIMS-ACTRIMS abstract EP1481]. Mult Scler J. 2017;23(suppl 3):777.

22. Benko AL, McAloose CA, Becker PM, Wright D, Sunyer T, Kawasawa YI, et al. Repository corticotrophin injection exerts direct acute effects on human B cell gene expression distinct from the actions of glucocorticoids. Clin Exp Immunol. 2018 Apr;192(1):68-81. https://doi.org/ 10.1111/cei.13089.

23. Wright D, Zweifel B, Prabha S, Galen K, Fitch R. Reduced steriodogenic activity of repository corticotropin injection (RCI) induces a distinct cytokine response following T cell activation [EULAR abstract AB0082]. Ann Rheum Dis. 2019b;78(suppl2):1504.

24. Huang YJ, Galen K, Zweifel B, Brooks LR, Wright AD. Distinct binding and signaling activity of Acthar Gel compared to other melanocortin receptor agonists. J Recept Signal Transduct. 2020:1-9. https://doi.org/ 10.1080/10799893.2020.1818094.

25. Schwyzer R, Schiller P, Seelig S, Sayers G. Isolated adrenal cells: log dose response curves for steroidogenesis induced by ACTH(1-24), ACTH(1-10), ACTH(4-10) and ACTH(5-10). FEBS Lett. 1971 Dec 15;19(3):229-31. https://doi.org/ 10.1016/0014-5793(71)80520-3.

26. Schwyzer R. ACTH: a short introductory review. Ann N Y Acad Sci. 1977 Oct 28;297:3-26. https://doi.org/10.1111/j.1749-6632.1977.tb41843.x.

27. Synacthen Depot [package insert]. Atnahs Pharma UK Limited; 2020.

28. Berg AL. Plasma cortisol levels after natural ACTH 1-39 and synthetic ACTH 1-24 in humans [NKF abstract 39]. Am J Kidney Dis. 2013;61(4):A25.. https://doi.org/ doi:10.1053/j.ajkd.2013.02.004.

29.Wang X, Pham L, Poola N, Brooks LR, Due B. Comparison of steroidogenic exposure following the administration of repository corticotropin injection with a synthetic ACTH1-24 depot and methylprednisolone in healthy subjects. Clin Pharmacol Drug Dev. 2020 Dec 23. https://doi.org/ 10.1002/cpdd.894.

30. Ponticelli C, Passerini P, Salvadori M, Manno C, Viola BF, Pasquali S, et al. A randomized pilot trial comparing methylprednisolone plus a cytotoxic agent versus synthetic adrenocorticotropic hormone in idiopathic membranous nephropathy. Am J Kidney Dis. 2006 Feb;47(2):233-40. https://doi.org/ 10.1053/j.ajkd.2005.10.016.

31. Nicolaides NC, Pavlaki AN, Alexandra M, Chrousos GP. Glucocorticoid therapy and adrenal suppression. In: Feingold KR, Anawalt B, Boyce A, et al., eds. Endotext [Internet]. MDText.com, Inc; 2018. Accessed May 20, 2020.

32. Paragliola RM, Papi G, Pontecorvi A, Corsello SM. Treatment with synthetic glucocorticoids and the hypothalamus-pituitary-adrenal axis. Int J Mol Sci. 2017 Oct 20;18(10). https://doi.org/ 10.3390/ijms18102201.

33. Coolens JL, Van Baelen H, Heyns W. Clinical use of unbound plasma cortisol as calculated from total cortisol and corticosteroid-binding globulin. J Steroid Biochem. 1987 Feb;26(2):197-202. https://doi.org/10.1016/0022-4731(87)90071-9.

34. Hangard G, Kirkeby K, Lingjaerde P. The potency of repository ACTH preparations. Acta Med Scand. 1960 Nov 1;168:205-10. https://doi.org/ 10.1111/j.0954-6820.1960.tb06663.x.

35. Brombacher PJ, Buytendijk HJ, Maesen F. Comparative trial with highly purified natural ACTH, synthetic ACTH 1-24 peptide and synthetic ACTH 1-39 peptide. Z Klin Chem Klin Biochem. 1969 May;7(3):291-2. https://doi.org/ 10.1515/cclm.1969.7.3.291.

36. Catania A, Lonati C, Sordi A, Carlin A, Leonardi P, Gatti S. The melanocortin system in control of inflammation. Sci World J. 2010 Sep 14;10:1840-53. https://doi.org/ 10.1100/tsw.2010.173.

37. Gaitonde SA, Gonzalez-Maeso J. Contribution of heteromerization to G protein-coupled receptor function. Curr Opin Pharmacol. 2017 Feb;32:23-31. https://doi.org/10.1016/j.coph.2016.10.006.

The present invention may also be described or defined in accordance with the following clauses:

1. A method of choosing an adrenal corticotropin treatment for a subject in need of such treatment, the method comprising (a) determining if a delayed corticosteroid response or a non-delayed response is appropriate for the subject; and (b) administering an adrenal corticotropin treatment consisting essentially of a non-synthetic adrenal corticotropin composition if a non-delayed response is appropriate or administering an adrenal corticotropin treatment consisting essentially of a synthetic adrenal corticotropin composition if a delayed response is appropriate.

2. The method of clause 1, wherein the corticosteroid response is a glucocorticoid steroid response.

3. The method of clause 2, wherein the glucocorticoid steroid response is a cortisol response.

4. The method of clause 1, wherein a delayed corticosteroid response results in a peak corticosteroid blood concentration greater than 8 hours after administration of the adrenal corticotropin treatment.

5. The method of clause 1, wherein a non-delayed corticosteroid response results in a peak corticosteroid blood concentration less than 8 hours after administration of the adrenal corticotropin treatment.

6. The method of clause 1, wherein the subject is diagnosed with an autoimmune disorder.

7. The method of clause 1, wherein the non-synthetic adrenal corticotropin composition comprises a respository corticotropin injection (RCI).

8. The method of clause 7, wherein the repository corticotropin injection is a naturally sourced complex mixture comprising N-25 deamidated porcine ACTH (1-39).

9. A method of designing a corticosteroid response in a subject, the method comprising: (a) selecting a subject in need of a designed corticosteroid response, (b) designing an appropriate corticosteroid response for the subject, and (c) administering one or more doses of an adrenal corticotropin treatment to the subject at a dose sufficient to invoke the desired corticosteroid response.

10. The method of clause 9, wherein the corticosteroid response is a glucocorticoid steroid response.

11. The method of clause 10, wherein the glucocorticoid steroid response is a cortisol response.

12. The method of clause 9, wherein the desired corticosteroid response peaks at between 2 and 8 hours post administration of an adrenal corticotropin treatment.

13. The method of clause 12, wherein the adrenal corticotropin treatment is a non-synthetic adrenal corticotropin treatment.

14. The method of clause 13, wherein the non-synthetic adrenal corticotropin composition comprises a respository corticotropin injection (RCI).

15. The method of clause 14, wherein the repository corticotropin injection is a naturally sourced complex mixture comprising N-25 deamidated porcine ACTH (1-39).

16. The method of clause 9, wherein the desired corticosteroid response is dose dependent.

17. The method of clause 16, wherein the adrenal corticotropin treatment is a non-synthetic adrenal corticotropin composition.

18. The method of clause 17, wherein the non-synthetic adrenal corticotropin composition comprises a respository corticotropin injection (RCI).

19. The method of clause 18, wherein the repository corticotropin injection is a naturally sourced complex mixture comprising N-25 deamidated porcine ACTH (1-39).

20. The method of clause 9, wherein the desired corticosteroid response is non-dose dependent.

21. The method of clause 20, wherein the adrenal corticotropin treatment consists essentially of a synthetic adrenal corticotropin composition.

22. A method of treating a subject, the method comprising: (a) determining if a strong corticosteroid response in the subject is beneficial; (b) when the answer to step (a) is no, administering one or more doses of repository corticotropin injection (RCI) to the subject at a dose sufficient to not invoke a strong corticosteroid response in the subject, wherein the desired corticosteroid response aids in the treatment of the subject.

23. The method of clause 22, wherein the corticosteroid response is a glucocorticoid steroid response.

24. The method of clause 23, wherein the glucocorticoid steroid response is a cortisol response.

25. The method of clause 22, wherein a strong corticosteroid response in the subject is beneficial when the subject is responsive to steroid therapy.

26. The method of clause 22, wherein the repository corticotropin injection is a naturally sourced complex mixture comprising N-25 deamidated porcine ACTH (1-39).

**Claims**

1. An adrenal corticotropin treatment for use in the treatment of a condition in a subject in need of such treatment, wherein the condition is susceptible to a corticosteroid response, the treatment comprising administering an adrenal corticotropin treatment consisting essentially of a non-synthetic adrenal corticotropin composition if a non-delayed corticosteroid response is determined to be appropriate in the subject, or administering an adrenal corticotropin treatment consisting essentially of a synthetic adrenal corticotropin composition if a delayed corticosteroid response is determined to be appropriate in the subject.

2. An adrenal corticotropin treatment for use in a method of designing a corticosteroid response in a subject, the method comprising: (a) selecting a subject in need of a designed corticosteroid response, (b) designing an appropriate corticosteroid response for the subject, and (c) administering one or more doses of an adrenal corticotropin treatment to the subject at a dose sufficient to invoke the desired corticosteroid response.

3. An adrenal corticotropin treatment for use in the treatment of a condition in a subject in need of such treatment, wherein the condition is susceptible to a corticosteroid response, the treatment comprising: administering, where it is determined that a strong corticosteroid response in the subject is not beneficial, one or more doses of repository corticotropin injection (RCI) to the subject at a dose sufficient to not invoke a strong corticosteroid response in the subject, wherein the desired corticosteroid response aids in the treatment of the subject.

4. The adrenal corticotropin treatment for use according to any of claims 1 to 3, wherein the corticosteroid response is a glucocorticoid steroid response.

5. The adrenal corticotropin treatment for use according to claim 4, wherein the glucocorticoid steroid response is a cortisol response.

6. The adrenal corticotropin treatment for use according to claim 1, wherein a delayed corticosteroid response results in a peak corticosteroid blood concentration greater than 8 hours after administration of the adrenal corticotropin treatment.

7. The adrenal corticotropin treatment for use according to claim 1, wherein a non-delayed corticosteroid response results in a peak corticosteroid blood concentration less than 8 hours after administration of the adrenal corticotropin treatment.

8. The adrenal corticotropin treatment for use according to any preceding claim, wherein the subject is diagnosed with an autoimmune disorder.

9. The adrenal corticotropin treatment for use according to claim 2, wherein the desired corticosteroid response peaks at between 2 and 8 hours post administration of an adrenal corticotropin treatment.

10. The adrenal corticotropin treatment for use according to claim 2 or claim 9, wherein the desired corticosteroid response is dose dependent.

11. The adrenal corticotropin treatment for use according to claim 9 or claim 10, wherein the adrenal corticotropin treatment is a non-synthetic adrenal corticotropin composition.

12. The adrenal corticotropin treatment for use according to any of claims 1, 10, and 11, wherein the non-synthetic adrenal corticotropin composition comprises a repository corticotropin injection (RCI).

13. The adrenal corticotropin treatment for use according to claim 3 or claim 12, wherein the repository corticotropin injection is a naturally sourced complex mixture comprising N-25 deamidated porcine ACTH (1-39).

14. The adrenal corticotropin treatment for use according to claim 2, wherein the desired corticosteroid response is non-dose dependent, and wherein, optionally, the adrenal corticotropin treatment consists essentially of a synthetic adrenal corticotropin composition.

15. The adrenal corticotropin treatment for use according to claim 3, wherein a strong corticosteroid response in the subject is beneficial when the subject is responsive to steroid therapy.

**Figure 1**

**Figure 2A**

**Figure 2B**

Figure B — Day 4: Baseline-corrected plasma free cortisol. Mean (SEM) baseline-corrected plasma free cortisol concentration (ng/mL) versus Time (hours postdose). Legend: Acthar Gel 80 IU (n=16); Synthetic ACTH$_{1-24}$ depot 1 mg (n=16). Shaded region: 0.2 to 23.0 ng/mL.

Figure 3A

EP 3 973 978 A1

**A**                    Day 1: Baseline-corrected serum total cortisol

**Figure 3B**

**Figure 4A and 4B**

**Figure 5A and 5B**

**Figure 6A and 6B**

| | Europäisches Patentamt European Patent Office Office européen des brevets | **EUROPEAN SEARCH REPORT** | | **Application Number** EP 21 19 6665 |
|---|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/150738 A1 (MALLINCKRODT ARD IP LTD [IE]; WRIGHT DALE [US]) 23 July 2020 (2020-07-23) *cf. sections [0001] to [0005] at pages 1 and 2, moreover sections [0009] to [0012], page 7, sections [0024] and [0025], sections [0084] to [0089] at pages 25-27, claims 1-22* | 1-15 | INV. A61K38/16 A61K38/35 A61P37/00 |
| E | US 2021/322520 A1 (WRIGHT DALE [US]) 21 October 2021 (2021-10-21) *cf. abstract, section [0003] at page 1, section [0019] at page 2, section [0033] at page 3, section [0098] at page 10, sections [0113] and [0114] at page 11 and 12, claims 1-26* | 1-15 | |

**TECHNICAL FIELDS SEARCHED (IPC)**

A61K
A61P

The present search report has been drawn up for all claims

| Place of search | Date of completion of the search | Examiner |
|---|---|---|
| **Munich** | **4 February 2022** | **Stoltner, Anton** |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 3 973 978 A1

## ANNEX TO THE EUROPEAN SEARCH REPORT
## ON EUROPEAN PATENT APPLICATION NO.

EP 21 19 6665

This annex lists the patent family members relating to the patent documents cited in the above-mentioned European search report.
The members are as contained in the European Patent Office EDP file on
The European Patent Office is in no way liable for these particulars which are merely given for the purpose of information.

04-02-2022

| Patent document cited in search report | | Publication date | Patent family member(s) | | Publication date |
|---|---|---|---|---|---|
| WO 2020150738 | A1 | 23-07-2020 | CA | 3125351 A1 | 23-07-2020 |
| | | | EP | 3911360 A1 | 24-11-2021 |
| | | | WO | 2020150738 A1 | 23-07-2020 |
| US 2021322520 | A1 | 21-10-2021 | NONE | | |

EPO FORM P0459

For more details about this annex : see Official Journal of the European Patent Office, No. 12/82

45

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020014444 W
- US 62794369
- US 63083532
- US 4554101 A
- US 4241046 A
- US 4394448 A
- US 4529561 A
- US 4755388 A
- US 4828837 A
- US 4925661 A
- US 4954345 A
- US 4957735 A
- US 5043164 A
- US 5064655 A
- US 5077211 A
- US 5264618 A

**Non-patent literature cited in the description**

- **KYTE et al.** *J. Mol. Biol.,* 1982, vol. 157, 105-132
- *Animal Genetics,* 2005, vol. 36, 160-190
- **CATANIA et al.** *Pharmacol. Rev.,* 2004, vol. 56, 1-29
- Remington's Pharmaceutical Sciences. 2005
- **GENNARO, A. R.** Remington's Pharmaceutical Sciences. Mack Publishing Co, 1995
- Pharmaceutical Dosage Forms. Marcel Dekker Inc, 1980
- **KODA-KIMBLE et al.** Applied Therapeutics: The Clinical Use of Drugs. Lippincott Williams & Wilkins, 2004
- **WINTER.** Basic Clinical Pharmacokinetics. Lippincott Williams & Wilkins, 2003
- **SHARQEL.** Applied Biopharmaceutics & Pharmacokinetics. McGraw-Hill/Appleton & Lange, 2004
- **MANIADAKIS N ; TOTH E ; SCHIFF M ; WANG X ; NASSIM M ; SZEGVARI B et al.** A targeted literature review examining biologic therapy compliance and persistence in chronic inflammatory diseases to identify the associated unmet needs, driving factors, and consequences. *Adv Ther,* September 2018, vol. 35 (9), 1333-55
- **SELMI C ; GENERALI E ; MASSAROTTI M ; BIANCHI G ; SCIRE CA.** New treatments for inflammatory rheumatic disease. *Immunol Res,* December 2014, vol. 60 (2-3), 277-88
- **WILLIAMS DM.** Clinical pharmacology of corticosteroids. *Respir Care,* June 2018, vol. 63 (6), 655-70
- **ZOOROB RJ ; CENDER D.** A different look at corticosteroids. *Am Fam Physician,* August 1998, vol. 58 (2), 443-50
- **WALJEE AK ; ROGERS MA ; LIN P ; SINGAL AG ; STEIN JD ; MARKS RM et al.** Short term use of oral corticosteroids and related harms among adults in the United States: population based cohort study. *BMJ,* April 2017, vol. 12 (357), j1415
- **BARNES PJ ; ADCOCK IM.** Glucocorticoid resistance in inflammatory diseases. *Lancet,* 30 May 2009, vol. 373 (9678), 1905-17
- **WILKINSON L ; VERHOOG NJD ; LOUW A.** Disease- and treatment-associated acquired glucocorticoid resistance. *Endocr Connect,* December 2018, vol. 7 (12), R328-R49
- **BOMBACK AS ; TUMLIN JA ; BARANSKI J ; BOURDEAU JE ; BESARAB A ; APPEL AS et al.** Drug Des Devel Ther. *Treatment of nephrotic syndrome with adrenocorticotropic hormone (ACTH) gel,* 14 March 2011, vol. 5, 147-53
- **BERKOVICH R ; AGIUS MA.** Mechanisms of action of ACTH in the management of relapsing forms of multiple sclerosis. *Ther Adv Neurol Disord,* March 2014, vol. 7 (2), 83-96
- **FIECHTNER JJ ; MONTROY T.** Treatment of moderately to severely active systemic lupus erythematosus with adrenocorticotropic hormone: a single-site, open-label trial. *Lupus,* August 2014, vol. 23 (9), 905-12
- **FURIE R ; MITRANE M ; ZHAO E ; DAS M ; LI D ; BECKER PM.** Efficacy and tolerability of repository corticotropin injection in patients with persistently active SLE: results of a phase 4, randomised, controlled pilot study. *Lupus Sci Med,* 2016, vol. 3 (1), e000180
- **MADAN A ; MIJOVIC-DAS S ; STANKOVIC A ; TEEHAN G ; MILWARD AS ; KHASTGIR A.** Acthar gel in the treatment of nephrotic syndrome: a multicenter retrospective case series. *BMC Nephrol,* 31 March 2016, vol. 17, 37

- **FURIE RA ; MITRANE M ; ZHAO E ; BECKER PM.** Repository corticotropin injection in patients with persistently active SLE requiring corticosteroids: post hoc analysis of results from a two-part, 52-week pilot study. *Lupus Sci Med,* 2017, vol. 4 (1), e000240
- **ALHAMAD T ; MANLLO DIECK J ; YOUNUS U ; MATAR D ; ALASFAR S ; VUJJINI V et al.** ACTH gel in resistant focal segmental glomerulosclerosis after kidney transplantation. *Transplantation,* January 2019, vol. 103 (1), 202-9
- **GRAFALS M ; SHARFUDDIN A.** Adrenocorticotropic hormone in the treatment of focal segmental glomerulosclerosis following kidney transplantation. *Transplant Proc,* 01 July 2019, vol. 51 (6), 1831-7
- **FLEISCHMANN R ; FURST DE ; CONNOLLY-STRONG E ; LIU J ; ZHU J ; BRASINGTON R.** Repository corticotropin injection for active rheumatoid arthritis despite aggressive treatment: a randomized controlled withdrawal trial. *Rheumatol Ther,* June 2020, vol. 7 (2), 327-44
- **ZAND L ; CANETTA P ; LAFAYETTE R ; ASLAM N ; JAN N ; SETHI S et al.** An open-label pilot study of adrenocorticotrophic hormone in the treatment of IgA nephropathy at high risk of progression. *Kidney Int Rep,* January 2020, vol. 5 (1), 58-65
- **ASKANASE AD ; ZHAO E ; ZHU J ; BILYK R ; FURIE RA.** Repository corticotropin injection for persistently active systemic lupus erythematosus: results from a phase 4, multicenter, randomized, double-blind, placebo-controlled trial. *Rheumatol Ther,* 29 September 2020, vol. 7 (4), 839-908
- **OLSEN NJ ; DECKER DA ; HIGGINS P ; BECKER PM ; MCALOOSE CA ; BENKO AL et al.** Direct effects of HP Acthar Gel on human B lymphocyte activation in vitro. *Arthritis Res Ther,* 27 October 2015, vol. 17, 300
- **HEALY LM ; JANG JH ; LIN YH ; RAO V ; ANTEL JP ; WRIGHT D.** Melanocortin receptor mediated anti-inflammatory effect of repository corticotropin injection on human monocyte-derived macrophages [ECTRIMS-ACTRIMS abstract EP1481. *Mult Scler J,* 2017, vol. 23 (3), 777
- **BENKO AL ; MCALOOSE CA ; BECKER PM ; WRIGHT D ; SUNYER T ; KAWASAWA YI et al.** Repository corticotrophin injection exerts direct acute effects on human B cell gene expression distinct from the actions of glucocorticoids. *Clin Exp Immunol,* April 2018, vol. 192 (1), 68-81
- **WRIGHT D ; ZWEIFEL B ; PRABHA S ; GALEN K ; FITCH R.** Reduced steriodogenic activity of repository corticotropin injection (RCI) induces a distinct cytokine response following T cell activation [EULAR abstract AB0082. *Ann Rheum Dis,* vol. 78 (2), 1504
- **HUANG YJ ; GALEN K ; ZWEIFEL B ; BROOKS LR ; WRIGHT AD.** Distinct binding and signaling activity of Acthar Gel compared to other melanocortin receptor agonists. *J Recept Signal Transduct,* 2020, 1-9
- **SCHWYZER R ; SCHILLER P ; SEELIG S ; SAYERS G.** Isolated adrenal cells: log dose response curves for steroidogenesis induced by ACTH(1-24), ACTH(1-10), ACTH(4-10) and ACTH(5-10). *FEBS Lett,* 15 December 1971, vol. 19 (3), 229-31
- **SCHWYZER R.** ACTH: a short introductory review. *Ann N Y Acad Sci,* 28 October 1977, vol. 297, 3-26
- **BERG AL.** Plasma cortisol levels after natural ACTH 1-39 and synthetic ACTH 1-24 in humans [NKF abstract 39. *Am J Kidney Dis,* 2013, vol. 61 (4), A25
- **WANG X ; PHAM L ; POOLA N ; BROOKS LR ; DUE B.** Comparison of steroidogenic exposure following the administration of repository corticotropin injection with a synthetic ACTH1-24 depot and methylprednisolone in healthy subjects. *Clin Pharmacol Drug Dev,* 23 December 2020
- **PONTICELLI C ; PASSERINI P ; SALVADORI M ; MANNO C ; VIOLA BF ; PASQUALI S et al.** A randomized pilot trial comparing methylprednisolone plus a cytotoxic agent versus synthetic adrenocorticotropic hormone in idiopathic membranous nephropathy. *Am J Kidney Dis,* February 2006, vol. 47 (2), 233-40
- **NICOLAIDES NC ; PAVLAKI AN ; ALEXANDRA M ; CHROUSOS GP et al.** Glucocorticoid therapy and adrenal suppression. MDText.com, Inc, 2018
- **PARAGLIOLA RM ; PAPI G ; PONTECORVI A ; CORSELLO SM.** Treatment with synthetic glucocorticoids and the hypothalamus-pituitary-adrenal axis. *Int J Mol Sci,* 20 October 2017, vol. 18 (10
- **COOLENS JL ; VAN BAELEN H ; HEYNS W.** Clinical use of unbound plasma cortisol as calculated from total cortisol and corticosteroid-binding globulin. *J Steroid Biochem,* February 1987, vol. 26 (2), 197-202
- **HANGARD G ; KIRKEBY K ; LINGJAERDE P.** The potency of repository ACTH preparations. *Acta Med Scand,* 01 November 1960, vol. 168, 205-10
- **BROMBACHER PJ ; BUYTENDIJK HJ ; MAESEN F.** Comparative trial with highly purified natural ACTH, synthetic ACTH 1-24 peptide and synthetic ACTH 1-39 peptide. *Z Klin Chem Klin Biochem,* May 1969, vol. 7 (3), 291-2
- **CATANIA A ; LONATI C ; SORDI A ; CARLIN A ; LEONARDI P ; GATTI S.** The melanocortin system in control of inflammation. *Sci World J.,* 14 September 2010, vol. 10, 1840-53

- **GAITONDE SA ; GONZALEZ-MAESO J.** Contribution of heteromerization to G protein-coupled receptor function. *Curr Opin Pharmacol,* February 2017, vol. 32, 23-31